# EUROPEAN PATENT SPECIFICATION

(11) **EP 2 285 956 B1**
(45) Date of publication and mention of the grant of the patent: **18.05.2016**
(21) Application number: 09762279.9
(22) Date of filing: 12.06.2009
(51) Int. Cl.: C12N 15/00

(54) **METHOD OF DIRECTING THE EVOLUTION OF AN ORGANISM**
VERFAHREN ZUR STEUERUNG DER EVOLUTION EINES ORGANISMUS
PROCÉDÉ D ORIENTATION DE L ÉVOLUTION D UN ORGANISME

(30) Priority: 13.06.2008 US 61485 P
(43) Date of publication of application: 23.02.2011
(73) Proprietor: Chitose Laboratory Corp., Miyamae-ku Kawasaki-shi Kanagawa 216-0001 (JP)
(72) Inventor: OGAWA, Masanori, Kawasaki-shi Kanagawa 216-0001 (JP); HORIUCHI, Takayuki, Kawasaki-shi Kanagawa 216-0001 (JP); TABATA, Kazufumi, Kawasaki-shi Kanagawa 216-0001 (JP); YANO, Shuntaro, Kawasaki-shi Kanagawa 216-0001 (JP); KUBOTA, Michi, Kawasaki-shi Kanagawa 216-0001 (JP); KURITA, Eri, Kawasaki-shi Kanagawa 216-0001 (JP); ITADANI, Akiko, Kawasaki-shi Kanagawa 216-0001 (JP); TANAKA, Sanae, Kawasaki-shi Kanagawa 216-0001 (JP)
(74) Representative: Lee, Nicholas John
(86) International application number: PCT/JP2009/002659
(87) International publication number: WO 2009/150848

(56) References cited:
- WO-A2-2004/087960
- US-A1- 2008 038 778
- MURPHY KELLY ET AL: "A method to select for mutator DNA polymerase delta s in Saccharomyces cerevisiae", GENOME, vol. 49, no. 4, April 2006 (2006-04), pages 403-410, XP002661227, ISSN: 0831-2796, DOI: 10.1139/G05-106
- BILES BENJAMIN D ET AL: "Low-fidelity Pyrococcus furiosus DNA polymerase mutants useful in error-prone PCR", NUCLEIC ACIDS RESEARCH, vol. 32, no. 22, E176, 2004, pages 1-7, XP002661228, ISSN: 0305-1048, DOI: 10.1093/nar/gnh174
- LONGLEY MATTHEW J ET AL: "The fidelity of human DNA polymerase gamma with and without exonucleolytic proofreading and the p55 accessory subunit", JOURNAL OF BIOLOGICAL CHEMISTRY, vol. 276, no. 42, 19 October 2001 (2001-10-19), pages 38555-38562, XP002661229, ISSN: 0021-9258, DOI: 10.1074/jbc.M105230200
- MCELHINNY STEPHANIE A NICK ET AL: "Inefficient proofreading and biased error rates during inaccurate DNA synthesis by a mutant derivative of Saccharomyces cerevisiae DNA polymerase delta", JOURNAL OF BIOLOGICAL CHEMISTRY, vol. 282, no. 4, 26 January 2007 (2007-01-26), pages 2324-2332, XP002661231, ISSN: 0021-9258, DOI: 10.1074/jbc.M609591200
- SHIMODA CHIKASHI ET AL: "Isolation of thermotolerant mutants by using proofreading-deficient DNA polymerase delta as an effective mutator in Saccharomyces cerevisiae", GENES & GENETIC SYSTEMS, vol. 81, no. 6, December 2006 (2006-12), pages 391-397, XP002661232, ISSN: 1341-7568
- FOSTER PATRICIA L ET AL: "Proofreading-defective DNA polymerase II increases adaptive mutation in Escherichia coli", PROCEEDINGS OF THE NATIONAL ACADEMY OF SCIENCES OF THE UNITED STATES OF AMERICA, vol. 92, no. 17, August 1995 (1995-08), pages 7951-7955, XP002661233, ISSN: 0027-8424
- BLAIR, M. M. ET AL.: 'DNA polymerase fidelity: a structural analysis' ACTA CRYST A. vol. A52, 1996, page C154, XP008145608
- GOLDSBY, R. E. ET AL.: 'High incidence of epithelial cancers in mice deficient for DNA polymerase delta proofreading.' PROC. NATL. ACAD. SCI. USA vol. 99, no. 24, 2002, pages 15560 - 15565, XP008145610
- VENKATESAN, R. N. ET AL.: 'Mutator phenotypes caused by substitution at a conserved motif A residue in eukaryotic DNA polymerase delta.' J. BIOL. CHEM. vol. 281, no. 7, 2006, pages 4486 - 4494, XP008145615
- SHEVELEV, I. V. ET AL.: 'The 3'-5' exonucleases.' NAT. REV. MOL. CELL BIOL. vol. 3, no. 5, 2002, pages 364 - 375, XP008098898

## Description

### Technical Field

The present disclosure relates to a method of directing the evolution of a Chinese hamster ovary cell by modifying the mutation rate of the cell.

### Summary of Invention

### Summary

The invention provides a method of directing the evolution of a Chinese hamster ovary (CHO) cell, the method comprising:
introducing at least one mutator gene into the CHO cell, wherein the at least one mutator gene comprises a low fidelity mutation and an exonuclease deficient mutation, wherein the introduction of the at least one mutator gene increases the mutation rate of the CHO cell;
growing the CHO cell into which the at least one mutator gene is introduced to obtain a grown cell; and
selecting at least one mutated cell with a desired hereditary trait from the grown cell,
wherein
(a) the exonuclease deficient mutation comprises a D398A (aspartic acid to alanine change at amino acid 398) amino acid substitution in at least one catalytic subunit of a DNA polymerase delta gene (Pold1) of the CHO cell; and
(b) the low fidelity mutation comprises a L602M (leucine to methionine change at amino acid 602) amino acid substitution in at least one catalytic subunit of a DNA polymerase delta gene (Pold1) of the CHO cell.

The method may further comprise restoring a wild-type mutation rate of the grown cell to obtain a restored grown cell, wherein the at least one mutated cell with a desired hereditary trait is selected from the restored grown cell. Alternatively, the method may further comprise restoring a wild-type mutation rate of the selected cell.

Modifying the mutation rate during DNA replication may increase the genetic diversity within and among individual organisms in a population. The increase in genetic diversity may be used to facilitate the selection of a desired hereditary trait in an organism. A desired trait may be selected by growing an organism with a modified mutation rate under selective conditions and isolating the individuals with the desired hereditary trait.

As described herein, the evolution of an organism can be directed by introducing one or more mutator genes or proteins into the organism. A gene or protein that controls, at least in part, DNA replication may be introduced into the organism to achieve a mutation rate that is higher than a wild-type mutation rate. For example, one or more mutator genes may be expressed within the organism in such a manner that results in an increased rate of mutation of the organism's DNA.

The one or more mutator genes may include one or more mutant DNA polymerases that are proofreading or exonuclease deficient relative to the wild-type allele. The one or more mutator genes may include one or more mutant DNA polymerases that exhibit a decreased polymerase fidelity relative to a wild-type allele. The one or more mutator genes expressed within the organism may include at least one mutant DNA polymerase that is exonuclease deficient and at least one mutant DNA polymerase that exhibits decreased polymerase fidelity relative to a wild-type allele.

The one or more mutator genes expressed within the organism may include at least one mutant DNA polymerase that is both exonuclease deficient and exhibits decreased polymerase fidelity relative to a wild-type allele.

Introduction of the one or more mutator genes as described herein does not involve substitution of one or more of an organism's endogenous DNA polymerase genes with a mutated copy. Even where introduction of the one or more mutator genes involves incorporation of such one or more genes into an organism's genome, the introduction is not targeted to replace or affect any of the organism's endogenous genes expressing a DNA polymerase.

The methods for directed evolution of an organism described herein may also include growing organisms having an increased mutation rate under conditions that exert selective pressure and selecting organisms having one or more desired traits. For example, the mutation rate of an organism may be modified to facilitate selection of an organism capable of growing under desired conditions, including, for example, the presence or absence of certain chemicals, nutrients, solvents or any other environmental conditions, including environmental conditions under which a wild-type organism would not grow or thrive. An organism having an increased mutation rate as described herein may be grown under conditions that result in an evolved organism that is resistant to attack or infection by another organism such as by a bacterial or viral pathogen. In a method of directed evolution as described herein, an organism having an increased mutation rate may be grown under conditions that exert selective pressure that results in an evolved organism that produces or processes a desired product, including, for example, particular oils, proteins, alcohols, or any other desired chemical product, more efficiently or in desired quantities.

Further, a method for directed evolution as described herein may include restoring the mutation rate exhibited by an organism having a modified mutation rate back to a wild-type mutation rate. In one such embodiment, once an organism having a desired trait is achieved and selected, the wild-type mutation rate may be restored by curing the evolved organism of the one or more mutator genes. Alternatively, the one or more mutator genes may be inducible under specific conditions, and restoration of the wild-type mutation rate can be achieved by subjecting the organism to conditions that do not result in transcription or translation of a mutator gene product. Even further, where one or more mutator genes included in an evolved and selected organism are incorporated into the genome of the evolved organism, restoration of the wild-type mutation rate may be achieved by excision or removal of said mutator gene from the genome or replacement of the mutator gene with a functional (non-mutator) allele.

The detailed description that follows also sets forth materials and organisms useful in carrying out the methods of directed evolution provided herein.

### Brief Description of Drawings

[fig.1A]FIG.1A shows the relative ethanol productivity of wild-type Taiken 396 parent yeast cells and transformed clotrimazole (CTZ) resistant yeast cells.
[fig.1B]FIG.1B compares the ethanol tolerance of a wild-type Taiken 396 parent yeast strain with the ethanol tolerance of transformed CTZ resistant yeast cells.
[fig.2]FIG.2 shows the growth of transformed tobacco cells on DBN herbicide growth medium.
[fig.3]FIG.3 shows the alignment of conserved exonuclease and polymerase motifs of DNA polymerase across multiple species.

### Description of Embodiments

Detailed Description

### Definitions

The term "evolution" as used herein refers to the occurrence of random variation in the genetic information passed from one individual to its descendants wherein the genetic variation may, or may not, be advantageous for survival and/or propagation of the organism. The evolution of an organism may be accelerated by increasing the occurrence of random variation in the genetic information of an organism. Populations with more genetic variation within and among individuals, may possess an increased ability to adapt to changing conditions such as environmental conditions and threats from pathogenic organisms.

The term "organism" as used herein refers to a body carrying on processes of life, which has various properties, such as, representatively, cellular structure, proliferation (self reproduction), growth, regulation, metabolism, repair ability, and the like. Typically, organisms possess basic attributes, such as heredity controlled by nucleic acids and proliferation in which metabolism controlled by proteins is involved. Organisms may include natural, wild-type, artificially manipulated, genetically modified, hybridized, or other variants or isolates. Organisms include viruses, prokaryotic organisms, eukaryotic organisms (e.g., unicellular organisms such as yeast, etc.) and multicellular organisms (e.g., plants, animals, etc.). It will be understood that, as the term is used herein, "organism" also refers to and encompasses cells as defined herein, and that the methods of the present disclosure may be applied to any such cell or cells.

The term "eukaryotic organism" as used herein refers to an organism having a clear nuclear structure with a nuclear envelope. Examples of eukaryotic organisms include, but are not limited to, unicellular organisms (e.g., yeast, etc.), plants (e.g., rice, wheat, maize, soybean, etc.), animals (e.g., mouse, rat, bovine, horse, swine, monkey, etc.), insects (e.g., fly, silkworm, etc.), and the like.

The term "unicellular organism" is used herein in its ordinary sense and refers to an organism consisting of one cell. Unicellular organisms include eukaryotic organisms. Examples of unicellular organisms include, but are not limited to, yeast, mammalian cells, cell cultures, and the like.

As used herein, the term "multicellular organism" refers to an individual organism consisting of a plurality of cells (typically, a plurality of cells of different types). Multicellular organisms include animals, plants, insects, and the like.

The term "animal" is used herein in its broadest sense and refers to vertebrates and invertebrates.

As used herein, the term "plant" refers to any organism belonging to the kingdom Plantae, including any of monocotyledonous plants and dicotyledonous plants. Examples of plants include, but are not limited to monocotyledonous plants of the rice family (e.g., wheat, maize, rice, barley, sorghum, etc.). Examples of preferable plants include tobacco, green pepper, eggplant, melon, tomato, sweet potato, cabbage, leek, broccoli, carrot, cucumber, citrus, Chinese cabbage, lettuce, peach, potato, and apple. Preferable plants are not limited to crops and include flowering plants, trees, lawn, weeds, and the like. Moreover, unless otherwise specified, the term "plant" refers to any of plant body, plant organ, plant tissue, plant cell, and seed. Examples of plant organ include root, leave, stem, flower, and the like. Examples of plant cells include, but are not limited to, callus, suspended culture cell, and the like.

As used herein, the term "hereditary trait", which is also called genotype, refers to a trait of an organism controlled by a gene.

As used herein, the term "gene" refers to a nucleic acid present in cells having a sequence of a predetermined length. A gene may or may not define a genetic trait. As used herein, the term "gene" typically refers to a sequence present in a genome and may refer to a sequence outside chromosomes, a sequence in mitochondria, or the like. A gene is typically arranged in a given sequence on a chromosome. A gene which defines the primary structure of a protein is called a structural gene. A gene which regulates the expression of a structural gene is called a regulatory gene (e.g., promoter). Genes herein include structural genes and regulatory genes unless otherwise specified. Therefore, for example, the term "DNA polymerase gene" typically refers to the structural gene of a DNA polymerase and its transcription and/or translation regulating sequences (e.g., a promoter). Regulatory sequences for transcription and/or translation as well as structural genes may be useful as genes targeted by the present disclosure. As used herein, "gene" may refer to "polynucleotide", "oligonucleotide", "nucleic acid", and "nucleic acid molecule" and/or "protein", "polypeptide", "oligopeptide" and "peptide". As used herein, "gene product" includes "polynucleotide", "oligonucleotide", "nucleic acid" and "nucleic acid molecule" and/or "protein", "polypeptide", "oligopeptide" and "peptide", which are expressed by a gene.

As used herein, the term "replication" in relation to a gene means that genetic material, DNA or RNA, reproduces a copy of itself, wherein a parent nucleic acid strand (DNA or RNA) is used as a template to form a new nucleic acid molecule (DNA or RNA, respectively) having the same structure and function as the parent nucleic acid. In eukaryotic cells, a replication initiating complex comprising a replication enzyme (DNA polymerase alpha) is formed to start replication at a number of origins of replication on a double-stranded DNA molecule, and replication reactions proceed in opposite directions from the origin of replication. The initiation of replication is controlled in accordance with a cell cycle. For example, in yeast, an autonomously replicating sequence may be regarded as an origin of replication. A replication initiating complex may be formed at the origin of replication. In one embodiment, the replication initiating complex can include a complex structure comprising one or more protein elements including a replication enzyme (DNA polymerase). In the replication reaction, the helical structure of double-stranded DNA may be partially rewound; a short DNA primer is synthesized; a new DNA strand is elongated from the 3'-OH group of the primer; Okazaki fragments are synthesized on a complementary strand template; the Okazaki fragments are ligated; proofreading is performed to compare the newly replicated strand with the template strand; and the like. Thus, the replication reaction may be performed via a number of reaction steps.

The replication mechanism of genomic DNA which stores the genetic information of an organism is described in detail in, for example, Kornberg A. and Baker T., "DNA Replication", New York, Freeman, 1992. Typically, an enzyme that uses one strand of DNA as a template to synthesize the complementary strand, forming a double-stranded DNA, is called DNA polymerase (DNA replicating enzyme). DNA replication requires at least two kinds of DNA polymerases. This is because typically, a leading strand and a lagging strand are simultaneously synthesized. DNA replication is started from a predetermined position of DNA, which is called an origin of replication (Ori). For example, bacteria have at least one bi-directional origin of replication on their circular genomic DNA. Thus, typically, four DNA polymerases need to simultaneously act on one genomic DNA during its replication. In one embodiment, replication error may be advantageously regulated on only one of a leading strand and a lagging strand, or alternatively, there may be advantageously a difference in the replication error or mutation rate between the two strands.

As used herein, the term "replication error" refers to introduction of an incorrect nucleotide during replication of a gene (DNA, etc.). Typically, the frequency of replication errors is as low as one in 10⁸ to 10¹² pairings. The reason the replication error frequency is low is at least two-fold: 1) nucleotide addition carried out by DNA polymerase is determined by complementary base pairing between template DNA and the nucleotides introduced during replication; and 2) the 3' to 5' exonuclease activity, or proofreading function, of DNA polymerase identifies and removes mispaired nucleotides which are not complementary to the template. Therefore, regulation of the mutation rate or the rate of DNA replication error in an organism can be carried out, for example, by altering or interrupting the fidelity with which a DNA polymerase forms correct nucleic acid base pairs and/or by altering the exonuclease function of a DNA polymerase.

As used herein, the term "DNA polymerase" or "Pol" refers to an enzyme which releases pyrophosphoric acid from deoxyribonucleoside 5'-triphosphate so as to polymerize DNA. DNA polymerase reactions require template DNA, a primer molecule, Mg ²⁺, and the like. Complementary nucleotides are sequentially added to the 3'-OH terminus of a primer to elongate a molecule chain.

As used herein, the term "proofreading function" refers to a function which detects and repairs a damage and/or an error in DNA of a cell. Such a function may be achieved by inserting bases at apurinic sites or apyrimidinic sites, or alternatively, cleaving one strand with an apurinic-apyrimidinic (A-P) endonuclease and then removing the sites with a 5' to 3' exonuclease. In the removed portion, DNA is synthesized and supplemented with a DNA polymerase, and the synthesized DNA is ligated with normal DNA by a DNA ligase. This reaction is called excision repair. For damaged DNA due to chemical modification by an alkylating agent, abnormal bases, radiation, ultraviolet light, or the like, the damaged portion is removed with a DNA glycosidase before repair is performed by the above-described reaction (unscheduled DNA synthesis). Examples of a DNA polymerase having such a exonuclease function include, but are not limited to, DNA polymerase delta, DNA polymerase epsilon, DNA polymerase gamma, etc.

A "proofreading deficient mutant" or "exonuclease deficient mutant" as used herein is intended to refer to a DNA polymerase mutant that has a 3' to 5' exonucleolytic proofreading activity that is lower than the 3' to 5' exonuclease activity of the selected parent polymerase from which the exonuclease deficient mutant is derived.

An "exonuclease function" as used herein refers to any of at least one of exonuclease functions, which includes proofreading, mismatch repair, Okazaki fragment maturation, and recombination. An "exonuclease deficient mutant", therefore, as used herein refers to a mutant that has impaired activity in any of at least one of these exonuclease functions.

As used herein, the term "fidelity" when used in reference to a DNA polymerase is intended to refer to the accuracy of nucleotide template-directed incorporation of complementary bases in a synthesized DNA strand relative to the template strand. Fidelity is measured based on the frequency of incorporation of incorrect bases in the newly synthesized nucleic acid strand. The incorporation of incorrect bases can result in point mutations, insertions or deletions. A polymerase fidelity mutant can exhibit either high fidelity or low fidelity. The term "low fidelity" is intended to mean a frequency of accurate base incorporation that is lower than a predetermined value. The predetermined value can be, for example, a desired frequency of accurate base incorporation or the fidelity of a known polymerase.

As used herein, the term "low fidelity mutant" refers to a polymerase mutant that has a DNA replication fidelity that is less than the replication fidelity of the selected parent polymerase from which the low fidelity mutant is derived. Altered fidelity can be determined by assaying the parent and mutant polymerase and comparing their activities using any assay that measures the accuracy of template directed incorporation of complementary bases.

As used herein, the term "mutation", means that a nucleotide sequence, such as the nucleotide sequence of a gene, is altered or refers to a state of the altered nucleic acid or the resulting amino acid sequence of the gene. For example, the term "mutation" herein refers to a change in the nucleotide sequence of a gene leading to a change in the resulting protein's function, such as a change in the exonuclease function of a polymerase.

The term "mutation" is used broadly herein and refers, for example, to point mutations, missense mutations, silent mutations, frame shift mutations, nonsense mutations, insertions or deletions of nucleotides, loss-of-function mutations, gain-of-function mutations, and dominant negative mutations. Mutations may be characterized as: A) neutral mutations which may have limited influence on the growth and metabolism of organisms; B) deleterious mutations which may inhibit the growth and metabolism of organisms; and C) beneficial mutations which can be beneficial for breeding of organisms.

As used herein, the term "growth" in relation to a certain organism refers to a quantitative increase in the individual organism. The growth of an organism can be recognized by a quantitative increase in a measured value, such as body size (body height), body weight, or the like. A quantitative increase in an individual depends on an increase in each cell and an increase in the number of cells.

As used herein the terms "wild-type mutation" and "spontaneous mutation" are used interchangeably. The rate of spontaneous mutation is defined as the probability of a mutation each time the genome is replicated or doubled. As used herein "mutation rate" is synonymous with "frequency" and refers to the absolute number of mutations/ doubling/base pair. As used herein, the term "relative rate" refers to the ratio of mutation rates of two organisms, one of these is usually a wild-type organism. Relative rate indicates how much more likely it is that an organism expressing a mutator gene will undergo mutation as compared to the wild-type organism. For example, the frequency of spontaneous mutation of wild-type E. coli (the E. coli genome has approximately 4.6x10⁶ base pairs) is approximately 5x10⁻¹⁰ mutations per base pair, per doubling.

As used herein, "mutator gene" refers to a gene which comprises a mutation that modifies the mutation rate of an organism. As used herein, the term "mutator plasmid" refers to a plasmid or expression vector or cassette comprising a mutator gene. Culturing an organism comprising a mutator gene may give rise to mutational events during genome replication. Mutator genes or mutator plasmids may comprise mutated DNA replication and/or DNA repair genes. DNA replication and repair genes include but are not limited to DNA polymerase I, DNA polymerase II, DNA polymerase III, Exo I, Exo II, Exo, III, Exo V, Exo VII, Exo IX, Exo X, RecJ, RnaseT, RnaseH, and homologues of these genes. Other mutator genes may include nucleases, 3'-5' exonucleases, 5'-3' exonucleases, DNA polymerase delta, DNA polymerase epsilon, Werner protein (WRN), p53, TREX1, TREX2, MRE11, RAD1, RAD9, APE1, VDJP, FEN1, and EXO1. A homologue as used herein refers to a functionally related gene.

The term "selected mutation" as used herein refers to those mutations which are associated with a phenotype of an evolved strain under a given set of conditions. "Being associated with" means that the mutation is directly or indirectly responsible for the improved or altered phenotype.

When referring to mutations or genetic changes in a host cell or organism, the term "nonspecific" refers to the changes in the host cell genome which occur randomly throughout the genome which potentially can affect all nucleotide bases and includes frameshifts. Nonspecific mutations encompass changes in single nucleotide base pairs as well as changes in multiple nucleotide base pairs as well as changes in large regions of DNA. For example, an organism which has been exposed to a gene comprising mutations that impair polymerase exonuclease function or fidelity may comprise non-specific, random mutations at a rate that is increased over wild-type.

When referring to genetic changes in a host cell, specific mutation refers to mutations which can be characterized by definable genetic changes, such as, without limitation, A:T to C:G transversions; G:C to T:A transversions; A:T to G:C and G:C to A:T transitions and frameshifts; and G:C to T:A transversions (Miller et al., A Short Course in Bacterial Genetics, a Laboratory Manual and Handbook for E. coli and Related Bacteria).

When referring to a mutator gene, "heterologous" means that the gene is introduced into the cell via recombinant methods known in the art. For example, the mutator gene may be introduced using a plasmid and may also be introduced into the microorganism genome. A mutator gene introduced into an organism may be a mutation of a naturally occurring endogenous DNA replication and repair gene in the cell or may be foreign to the host microorganism. Referring to nucleic acid as being "introduced" into a microorganism means that the nucleic acid is inserted into the microorganism using standard molecular biology techniques. An introduced nucleic acid may be the same or different than nucleic acid naturally occurring in the microorganism.

As used herein the term "restoring to wild-type mutation rate" refers to a process whereby a mutator gene is removed from an organism or disabled, thereby restoring the wild-type mutation rates. The present disclosure encompasses any process for removing a mutator gene from an evolved organism and includes but is not limited to curing the organism of a resident plasmid comprising a mutator gene or by excising or otherwise removing a mutator gene from the host genome such that normal DNA replication and repair function is restored. Curing refers to methods for producing cells which are free of a plasmid or other vector comprising the mutator gene. An organism can be cured of any resident plasmid or other vector using techniques known to the skilled artisan.

As used herein, the term "reproduction" in relation to an organism means that a new individual of the next generation is produced from a parent individual. Reproduction includes, but is not limited to, natural multiplication, proliferation, and the like; artificial multiplication, proliferation, and the like by artificial techniques, such as cloning techniques (nuclear transplantation, etc.). Examples of a technique for reproduction include, but are not limited to, culturing of a single cell, grafting of a cutting, rooting of a cutting, and the like, in the case of plants. Reproduced organisms typically have hereditary traits derived from their parents. Sexually reproduced organisms have hereditary traits derived from typically two sexes. Typically, these hereditary traits are derived from two sexes in substantially equal proportions. Asexually reproduced organisms have hereditary traits derived from their parents.

The term "cell" is herein used in its broadest sense. Cells may be naturally-occurring cells or artificially modified cells (e.g., fusion calls, genetically modified cells, etc.). Cells may be derived from any organism (e.g., any unicellular organism such as bacteria, yeast, animal cells, plant cells, cell cultures, etc.) or any multicellular organism (e.g., animals, plants, etc.). Examples of a source for cells include, but are not limited to, a single cell culture, the embryo, blood, or body tissue of a normally grown transgenic animal, a cell mixture, such as cells from a normally grown cell line, and the like.

A "cell", as used herein, may be grown and allowed to differentiate to form one or more multicellular organisms after being selected for at least one desired trait of the cell. Such multicellular organisms grown from the cell may have the same desired trait as a whole when compared to the cell.

As used herein, the term "isolated" indicates that at least a naturally accompanying substance in a typical environment is reduced, preferably substantially excluded. Therefore, the term "isolated cell" refers to a cell which contains substantially no naturally accompanying substance in a typical environment (e.g., other cells, proteins, nucleic acids, etc.). The term "isolated" in relation to a nucleic acid or a polypeptide refers to a nucleic acid or a polypeptide which contains substantially no cellular substance or culture medium when is produced by recombinant DNA techniques or which contains substantially no precursor chemical substance or other chemical substances when it is chemically synthesized, for example. Preferably, isolated nucleic acids do not contain a sequence which naturally flanks the nucleic acid in organisms (the 5' or 3' terminus of the nucleic acid).

As used herein, the term "differentiated cell" refers to a cell having a specialized function and form (e.g., muscle cells, neurons, etc.). Unlike stem cells, differentiated cells have no or little pluripotency. Examples of differentiated cells include epidermic cells, pancreatic parenchymal cells, pancreatic duct cells, hepatic cells, blood cells, cardiac muscle cells, skeletal muscle cells, osteoblasts, skeletal myoblasts, neurons, vascular endothelial cells, pigment cells, smooth muscle cells, fat cells, bone cells, cartilage cells.

As used herein, the terms "differentiation" or "cell differentiation" refers to a phenomenon that two or more types of cells having qualitative differences in form and/ or function occur in a daughter cell population derived from the division of a single cell. Therefore, "differentiation" includes a process during which a population (family tree) of cells which do not originally have a specific detectable feature acquire a feature, such as production of a specific protein.

As used herein, the term "tissue" refers to an aggregate of cells having substantially the same function and/or form in a multicellular organism. A tissue is typically an aggregate of cells of the same origin, but may be an aggregate of cells of different origins as long as the cells have the same function and/or form. Typically, a tissue constitutes a part of an organ.

Any organ or a part thereof may be used in the various embodiments of the disclosure. Likewise, tissues or cells may be derived from any organ. As used herein, the term "organ" refers to a morphologically independent structure localized at a particular portion of an individual organism in which a certain function is performed. In multicellular organisms (e.g., animals, plants), an organ consists of several tissues spatially arranged in a particular manner, each tissue being composed of a number of cells. An example of such an organ includes an organ relating to the vascular system. In one embodiment, organs targeted by the present disclosure may include, but are not limited to, skin, blood vessel, cornea, kidney, heart, liver, umbilical cord, intestine, nerve, lung, placenta, pancreas, brain, peripheral limbs, retina, and the like.

As used herein, the term "product" refers to a substance produced by an organism of interest or a part thereof. Examples of such a product substance include, but are not limited to, expression products of genes, metabolites, excrements, proteins, chemicals, antibodies, alcohols, enzymes, and the like. According to one embodiment, regulating the mutation rate of a hereditary trait, an organism of interest may be allowed to change the type and/or amount of a product.

The terms "protein", "polypeptide", "oligopeptide" and "peptide" as used herein have the same meaning and refer to an amino acid polymer having any length. This polymer may be a straight, branched or cyclic chain. An amino acid may be a naturally-occurring or non-naturally-occurring amino acid, or a variant amino acid. The term may include those assembled into a complex of a plurality of polypeptide chains. The term also includes a naturally-occurring or artificially modified amino acid polymer. Such modification includes, for example, disulfide bond formation, glycosylation, lipidation, acetylation, phosphorylation, or any other manipulation or modification (e.g., conjugation with a labeling moiety). This definition encompasses a polypeptide containing at least one amino acid analog (e.g., non-naturally-occuring amino acid, etc.), a peptide-like compound (e.g., peptoid), and other variants known in the art for example. In one embodiment, a gene product may be in the form of a polypeptide and may be useful as a pharmaceutical composition.

The terms "polynucleotide", "oligonucleotide" and "nucleic acid" as used herein have the same meaning and refer to a nucleotide polymer having any length including cDNA, mRNA, and genomic DNA. As used herein, nucleic acid and nucleic acid molecule may be included by the concept of the term "gene." A nucleic acid molecule encoding the sequence of a given gene includes "splice mutant (variant)". Similarly, a particular protein encoded by a nucleic acid encompasses any protein encoded by a splice variant of that nucleic acid. "Splice mutants", as the name suggests, are products of alternative splicing of a gene. After transcription, an initial nucleic acid transcript may be spliced such that different (alternative) nucleic acid splice products encode different polypeptides. Mechanisms for the production of splice variants vary, but include alternative splicing of exons. Alternative polypeptides derived from the same nucleic acid by read-through transcription are also encompassed by this definition. Any products of a splicing reaction, including recombinant forms of the splice products, may be included in this definition. Therefore, a gene may include the splice mutants herein.

Unless otherwise indicated, a particular nucleic acid sequence also implicitly encompasses conservatively-modified variants thereof (e.g. degenerate codon substitutions) and complementary sequences as well as the sequence explicitly indicated. Specifically, degenerate codon substitutions may be produced by generating sequences in which the third position of one or more selected (or all) codons is substituted with mixed-base and/or deoxyinosine residues (Batzer et al., Nucleic Acid Res. 19:5081(1991); Ohtsuka et al., J. Biol. Chem. 260:2605-2608 (1985); Rossolini et al., Mol. Cell. Probes 8:91-98(1994)).

As used herein, "homology" of a gene (e.g., a nucleic acid sequence, an amino acid sequence, or the like) refers to the proportion of identity between two or more gene sequences. As used herein, the identity of a sequence (a nucleic acid sequence, an amino acid sequence) refers to the proportion of the identical sequence (an individual nucleic acid, amino acid) between two or more comparable sequences. Therefore, the greater the homology between two given genes, the greater the identity or similarity between their sequences. Whether or not two genes have homology is determined by comparing their sequences directly or by a hybridization method under stringent conditions. When two gene sequences are directly compared with each other, these genes have homology if the DNA sequences of the genes have representatively at least 50% identity, at least 70% identity, at least 80%, 90%, 95%, 96%, 97%, 98%, or 99% identity with each other. As used herein, "similarity" of a gene (e.g. a nucleic acid sequence, an amino acid sequence, or the like) refers to the proportion of identity between two or more sequences when conservative substitution is regarded as positive (identical) in the above-described homology. Therefore, homology and similarity differ from each other in the presence of conservative substitutions. If no conservative substitutions are present, homology and similarity have the same value.

As used herein, the term "primer" refers to a substance required for initiation of a reaction of a macromolecule compound to be synthesized, in a macromolecule synthesis enzymatic reaction. In a reaction for synthesizing a nucleic acid molecule, a nucleic acid molecule (e.g., DNA, RNA, or the like) which is complementary to part of a macromolecule compound to be synthesized may be used.

A nucleic acid molecule which is ordinarily used as a primer includes one that has a nucleic acid sequence having a length of at least 8 contiguous nucleotides, which is complementary to the nucleic acid sequence of a gene of interest. Such a nucleic acid sequence preferably has a length of at least 9 contiguous nucleotides, more preferably a length of at least 10 contiguous nucleotides, even more preferably a length of at least 11 contiguous nucleotides, a length of at least 12 contiguous nucleotides, a length of at least 13 contiguous nucleotides, a length of at least 14 contiguous nucleotides, a length of at least 15 contiguous nucleotides, a length of at least 16 contiguous nucleotides, a length of at least 17 contiguous nucleotides, a length of at least 18 contiguous nucleotides, a length of at least 19 contiguous nucleotides, a length of at least 20 contiguous nucleotides, a length of at least 25 contiguous nucleotides, a length of at least 30 contiguous nucleotides, a length of at least 40 contiguous nucleotides, and a length of at least 50 contiguous nucleotides. A nucleic acid sequence used as a primer includes a nucleic acid sequence having at least 70% homology to the above-described sequence, more preferably at least 80%, even more preferably at least 90%, and most preferably at least 95%. An appropriate sequence as a primer may vary depending on the property of the sequence to be synthesized (amplified). Those skilled in the art can design an appropriate primer depending on the sequence of interest. Such a primer design is well known in the art and may be performed manually or using a computer program.

As used herein, the term "variant" refers to a substance, such as a polypeptide or polynucleotide, which differs partially from the original substance. Examples of such a variant include a substitution variant, an addition variant, a deletion variant, a truncated variant, an allelic variant, and the like. Examples of such a variant include, but are not limited to, a nucleotide or polypeptide having one or several substitutions, additions and/or deletions or a nucleotide or polypeptide having at least one substitution, addition and/or deletion with respect to a reference nucleic acid molecule or polypeptide. Variant may or may not have the biological activity of a reference molecule (e.g., a wild-type molecule, etc.). Variants may be conferred additional biological activity, or may lack a part of biological activity, depending on the purpose. Such design can be carried out using techniques well known in the art. Alternatively, variants, whose properties are already known, may be obtained by isolation from organisms to produce the variants and the nucleic acid sequence of the variant may be amplified so as to obtain the sequence information. Therefore, for host cells, corresponding genes derived from heterologous species or products thereof are regarded as "variants".

The terms "nucleic acid molecule" as used herein includes one in which a part of the naturally occurring sequence of the nucleic acid is deleted or is substituted with other base(s), or an additional nucleic acid sequence is inserted, as long as a polypeptide expressed by the nucleic acid has substantially the same activity as that of the naturally-occurring polypeptide, as described above. Alternatively, an additional nucleic acid may be linked to the 5' terminus and/or 3' terminus of the nucleic acid. The nucleic acid molecule may include one that may hybridize to a gene encoding a polypeptide under stringent conditions and encodes a polypeptide having substantially the same function. A nucleic acid can be obtained by a well-known PCR method, i.e., chemical synthesis. This method may be combined with, for example, site-directed mutagenesis and hybridization.

As used herein, the term "substitution", "addition" or "deletion" for a polypeptide or a polynucleotide refers to the substitution, addition or deletion of an amino acid or its substitute, or a nucleotide or its substitute, with respect to the original polypeptide or polynucleotide, respectively. This is achieved by techniques well known in the art, including a site-directed mutagenesis technique. A polypeptide or a polynucleotide may have any number (>0) of substitutions, additions, or deletions. The number can be as large as a variant having such a number of substitutions, additions or deletions which maintains an intended function (e.g., the information transfer function of hormones and cytokines, etc.). For example, such a number may be one or several, and preferably within 20% or 10% of the full length, or no more than 100, no more than 50, or no more than 25.

The term "vector" or "recombinant vector" or "expression vector" or "mutator vector" refers to a vector capable of transferring a polynucleotide sequence of interest to a target cell. Such a vector may be capable of self-replication or incorporation into a chromosome in a host cell (e.g., yeast, an animal cell, a plant cell, an insect cell, an individual animal, and an individual plant, etc.), and contains a promoter at a site suitable for transcription of a polynucleotide. A vector suitable for cloning is referred to as "cloning vector". Such a cloning vector ordinarily contains a multiple cloning site containing a plurality of restriction sites. Restriction sites and multiple cloning sites are well known in the art and may be appropriately or optionally used depending on the purpose. Such vectors include, for example, plasmids and viral vectors. It is well known to those skilled in the art that the type of organism (e.g., a plant) expression vector and the type of regulatory element may vary depending on the host cell.

Vectors may include integration-type vectors that can introduce a nucleotide sequence into the genome of the host by site-specific recombination or random insertion. The integration-type vector may use homologous recombination to insert the mutator gene into a genome target site, such as a DNA polymerase gene. Integration-type vector may include entire mutator genes, mutator gene fragments, and mutator genes with flanking nucleotides that may be used to enable homologous recombination.

Other vectors may be inducible vectors that include inducible systems that can regulate expression of a transgene in the host organism. Inducible vectors may include a galactose-inducible GAL1:URA3 expression system, Cre/loxP system or a tetracycline-inducible expression system.

As used herein, the term "plasmid" refers to a hereditary factor which is present apart from chromosomes and autonomously replicates. When specifically mentioned, DNA contained in mitochondria and chloroplasts of cell nuclei is generally considered organelle DNA and is distinguished from plasmids, i.e., is not included in within the definition of the term "plasmid" as used herein. Plasmid vectors may consist of an origin of replication that allows for semi-independent replication of the plasmid in the host. Plasmids may vary in their copy number depending on the origin of replication which they contain which determines whether they are under relaxed or stringent control. Some plasmids are high-copy plasmids and have mutations which allow them to reach very high copy numbers within the host cell. Other plasmids may be low-copy plasmids and are often maintained at very low copy numbers per cell.

As used herein, the term "promoter" refers to a base sequence which determines the initiation site of transcription of a gene and is a DNA region which directly regulates the frequency of transcription. Transcription is started by RNA polymerase binding to a promoter. Therefore, a portion of a given gene which functions as a promoter is herein referred to as a "promoter portion". A promoter region is usually located within about 2 kbp upstream of the first exon of a putative protein coding region. Therefore, it is possible to estimate a promoter region by predicting a protein coding region in a genomic base sequence using DNA analysis software. A putative promoter region is usually located upstream of a structural gene, but depending on the structural gene, i.e., a putative promoter region may be located downstream of a structural gene. By placing a gene under control of a specific promoter expression of such gene can be regulated under certain conditions.

Molecular biological techniques, biochemical techniques, microorganism techniques, and cellular biological techniques as used herein are well known in the art and commonly used, and are described in, for example, Sambrook, J. et al. (1989), Molecular Cloning: A Laboratory Manual, Cold Spring Harbor, and its 3rd Ed. (2001); Ausubel, F. M. (1987), Current Protocols in Molecular Biology, Greene Pub. Associates and Wiley-Interscience; Ausubel, F. M. (1989), Short Protocols in Molecular Biology: A Compendium of Methods from Current Protocols in Molecular Biology, Greene Pub. Associates and Wiley-Interscience; Innis, M. A. (1990), PCR Protocols: A Guide to Methods and Applications, Academic Press; Ausubel, F. M. (1992), Short Protocols in Molecular Biology: A Compendium of Methods from Current Protocols in Molecular Biology, Greene Pub. Associates; Ausubel, F. M. (1995), Short Protocols in Molecular Biology: A Compendium of Methods from Current Protocols in Molecular Biology, Greene Pub. Associates; Innis, M. A. et al. (1995), PCR Strategies, Academic Press; Ausubel, F. M. (1999), Short Protocols in Molecular Biology: A Compendium of Methods from Current Protocols in Molecular Biology, Wiley, and annual updates; Sninsky, J. J. et al. (1999), PCR Applications: Protocols for Functional Genomics, Academic Press; Special issue, Jikken Igaku, (Experimental Medicine), Indenshi Donyu & Hatsugen Kaiseki Jikkenho, (Experimental Methods for Gene introduction & Expression Analysis), Yodo-sha, (1997).

DNA synthesis techniques and nucleic acid chemistry for preparing artificially synthesized genes are described in, for example, Gait, M. J. (1985), Oligonucleotide Synthesis: A Practical Approach, IRL Press; Gait, M. J. (1990), Oligonucleotide Synthesis: A Practical Approach, IRL Press; Eckstein, F. (1991), Oligonucleotides and Analogues: A Practical Approach, IRL Press; Adams, R. L. et al. (1992), The Bio-chemistry of the Nucleic Acids, Chapman & Hall Shabarova, Z. et al. (1994), Advanced Organic Chemistry of Nucleic Acids, Weinheim; Blackburn, G. M. et al. (1996), Nucleic Acids in Chemistry and Biology, Oxford University Press; Hermanson, G. T. (1996), Bioconjugate techniques, Academic Press.

### Methods for Directing Evolution

A method for directing evolution of an organism according to the present description includes modifying the mutation rate of the organism. In particular, modifying the mutation rate of the organism as described herein includes introducing one or more mutator genes into the organism in a manner that results in expression of the one or more mutator genes and an increase in the DNA mutation rate relative to the parent organism. The one or more mutator genes may include a mutant DNA polymerase the expression of which alters the DNA polymerase function of the organism. The mutant DNA polymerase may be a low fidelity mutant. The mutant DNA polymerase may be an exonuclease deficient mutant. The one or more mutator genes may include at least one mutant DNA polymerase that is a low fidelity mutant and at least one DNA polymerase that is an exonuclease deficient mutant.

Introducing one or more mutator genes into the organism may include introducing at least one mutant DNA polymerase that is both a low fidelity mutant and an exonuclease deficient mutant.

Various different DNA polymerases are known in the art. Based on differences in the primary structure of their catalytic subunits, DNA polymerases are classified into several distinct families. Family A is named after the E. coli polA gene that encodes Pol I. Family A members also include the bacteriophage T7 replicative polymerase and eukaryotic mitochondrial polymerase gamma, as well as human polymerases Pol theta, and Pol nu. Family B polymerases include E. coli Pol II, the product of the polB gene, and the eukaryotic polymerases alpha, delta, epsilon, zeta. Family C polymerases include the E. coli replicative polymerase Pol III, whose catalytic subunit is encoded by the polC gene.

E. coli possesses at least three DNA polymerases, DNA polymerases I, II, and III. DNA polymerase I is involved in DNA repair, recombination, and Okazaki fragment maturation. DNA polymerase II is involved in DNA repair. DNA polymerase III is involved in chromosomal DNA replication and repair. These polymerase enzymes each have a subunit structure comprising several proteins and are divided into a core enzyme or a holoenzyme in accordance with the structure. A core polymerase enzyme is composed of alpha, epsilon, and theta subunits. A holoenzyme comprises tau, gamma, delta, and beta components in addition to alpha, epsilon, and theta subunits.

Eukaryotic cells have a plurality of DNA polymerases including, without limitation, DNA polymerases alpha, beta, delta, gamma, and epsilon. Known polymerases in animals include DNA polymerase alpha which may be involved in replication of nuclear DNA and may play a role in DNA replication in a cell growth phase. DNA polymerase beta may be involved in DNA repair in nuclei and repair of damaged DNA in the growth phase and the quiescent phase. DNA polymerase gamma may be involved in replication and repair of mitochondrial DNA and has exonuclease activity. DNA polymerase delta may be involved in replication of nuclear DNA and may play a role in DNA elongation of lagging strand and also includes exonuclease activity. DNA polymerase epsilon may be involved in replication of nuclear DNA and may play a role in DNA elongation of leading strand and has exonuclease activity.

The exonuclease function of DNA polymerase in gram-positive bacteria, gram-negative bacteria, and eukaryotic organisms likely includes amino acid sequences having an Exo I motif with a role in the 3' to 5' exonuclease activity center which may have an influence on the accuracy of the exonuclease function. In gram-negative bacteria, such as E. coli, there are two DNA polymerase proteins, i.e., a molecule having exonuclease activity and a molecule having DNA synthesis activity. However, in gram-positive bacteria as well as eukaryotic organisms, a single DNA polymerase has both DNA synthesis activity and exonuclease activity.

The DNA polymerase may also include fidelity functions of the polymerase active site which involve the ability of a polymerase to select a correct nucleotide during DNA replication. DNA polymerase active sites include, for example, regions of the Pol delta catalytic subunits.

DNA polymerase variants have been found in prokaryotic and eukaryotic organisms. A number of replicative DNA polymerases have a proofreading or exonuclease function, i.e., remove errors by 3' to 5' exonuclease activity to perform error-free replication. Error-prone DNA polymerase variants may have damaged or malfunctioning exonuclease functions, which may result in nucleotide sequence mutations during replication. Alternatively, error-prone DNA polymerase variants may be low fidelity mutants that exhibit a lower-than-wild-type replication fidelity, which also may result in nucleotide sequence mutations during replication. Any DNA polymerase that may become error-prone by modifying either or both of its exonuclease function and/or the fidelity with which it creates complementary nucleotide base pairs may be used as a mutator gene in the methods for directed evolution described herein. A mutant DNA polymerase introduced into an organism according to the present description may be a heterologous DNA polymerase, a mutant heterologous DNA polymerase or a mutant version of a DNA polymerase endogenous to the organism. Moreover, the modifications necessary to achieve a mutant DNA polymerase as described herein may be carried out by biological techniques well known in the art.

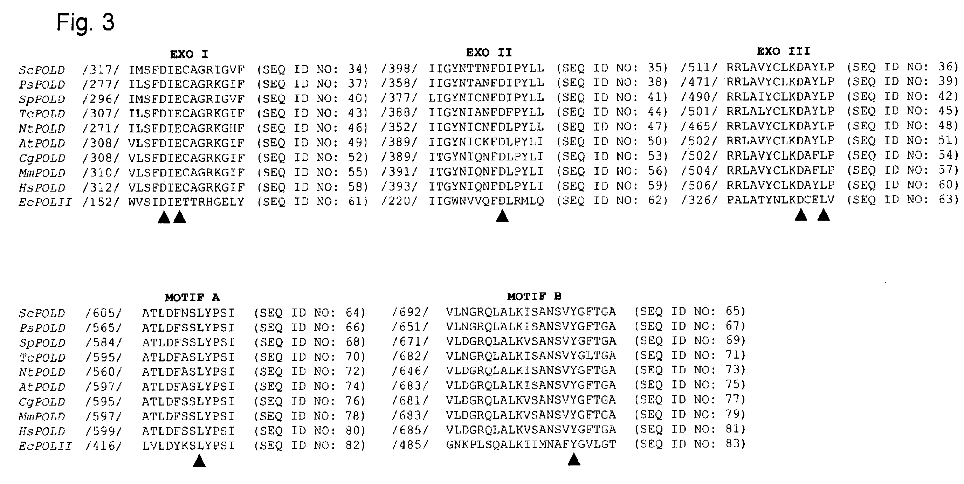

An exonuclease deficient mutant DNA polymerase may be created, for example, by introducing one or more mutations in the gene encoding the DNA polymerase that modify the 3' to 5' exonuclease activity of the polymerase, such as, for example by modifying an Exo I, Exo II, and/or Exo III motif or other exonuclease function active site of the Pol delta, Pol epsilon, and/or Pol gamma DNA polymerases. Exemplary mutations that result in exonuclease deficient mutant DNA polymerases are described in detail in association with the experimental examples provided herein. Additional mutations that lead to exonuclease deficient mutant DNA polymerases are described in, for example, Shevelev, IV and U. Hubscher, "The 3' 5' exonucleases", Nat. Rev. Mol. Cell Biol., 2002 May; 3(5):364-76 . Furthermore, as shown in FIG. 3, the exonuclease domains Exo I, Exo II, and Exo III of DNA polymerases, such as those shown by the arrows, are conserved across multiple species such as the yeast S. cerevisiae (Sc), the yeast Pichia stipitis (Ps), the yeast Schizosaccharomyces pombe (Sp), the fungi Tolypocladium inflatum (Tc), the tobacco plant Nicotiana tobacum (Nt), the plant Arabidopsis thaliana (At), the Chinese hamster Cricetulus griseus (Cg), the mouse Mus musculus (Mm), Homo sapiens (Hs), and the bacteria Escherichia coli (Ec).

Exemplary mutations that result in low fidelity DNA polymerases are described in detail in association with the experimental examples provided herein. In one embodiment, a mutation resulting in decreased substrate recognition by the DNA polymerase may be considered as polymerase low fidelity mutation. More particularly, one or more mutations at substrate recognition sites and/or active sites may be examples of polymerase low fidelity mutations. Substrate recognition sites of DNA polymerase are described in, for example, Reha-Krantz and Nonay, "Motif A of Bacteriophage T4 DNA polymerase: Role in Primer Extension and DNA Replication Fidelity", J. Biol. Chem., 1994 Feb. 25; 269(8): 5635-43, Li et al., "Sensitivity to Phosphonoacetic Acid: A New Phenotype to Probe DNA Polymerase delta in Saccharomyces cerevisiae", Genetics, 2005 Jun. ; 170: 569-580, Venkatesan et al., "Mutator phenotypes caused by substitution at a conserved motif A residue in eukaryotic DNA polymerase delta", J. Biol. Chem., 2006 Feb. 17; 281(7): 4486-94, Pursell et al., "Regulation of B family DNA polymerase fidelity by a conserved active site residue: characterization of M644W, M644L and M644F mutants of yeast DNA polymerase epsilon", N.A.R. 2007 Apr. 22; 35 (9): 3076-86, McElhinny et al., "Inefficient Proofreading and Biased Error Rates during Inaccurate DNA Synthesis by a Mutant Derivative of Saccharomyces cerevisiae DNA polymerase delta" J. Biol. Chem., 2007 Jan. 26; 282(4): 2324-32.

The mutator gene may include mutations that impair exonuclease function of the Exo I motif of DNA polymerase in yeast, or the Exo II motif of DNA polymerase in CHO cells by decreasing the 3' to 5' exonuclease activity.

The mutator gene may include mutations that impair the base pair matching fidelity of the Motif A subunit or the Motif B subunit of DNA polymerase.

The mutator gene can include one or more mutations that may manipulate the function of a DNA polymerase B gene (PoIII) in E. coli.

The mutator gene can include one or more mutations that may alter the function of a DNA polymerase III gene in B. subtilis. As will be apparent from these examples, amino acid residues that are essential and responsible for 3' to 5' exonuclease activity and for polymerase fidelity are preserved across polymerases belonging to different families.

A mutant DNA polymerase used as a mutator gene in a method as described herein may create a particular mutation rate in the target organism. For example, a mutator gene used in a method for directed evolution as described herein may be a DNA polymerase that is both a low fidelity mutant and a exonuclease deficient mutant that creates more than one mutation per generation or cell division.

The mutator gene may cause at least 2, 3, 4, 5, 6, 7, 8, 9, or 10 mismatched bases, or at least 15, 20, 25, 50, and 100 mismatched bases per DNA replication. Alternatively, a mutator gene used in a method for directed evolution as described herein may be a DNA polymerase that is both a low fidelity mutant and a exonuclease deficient mutant that leads to mutation rate in the organism ranging from approximately a 2-fold increase, to at most a 100,000-fold increase in the mutation rate relative to the parent strain. In one such example , the mutator gene confers a mutation rate increase ranging from approximately 2-fold to approximately 1,000 fold greater than the mutation rate of the parent strain. In another example, the mutator plasmid confers an increased mutation rate of approximately 10, 20, 30, 40, 50, 60, 70, 80, 100, 120, 140, 160, 170, 190, 200, 250, 300, 350, and 400-fold greater than the mutation rate of the parent strain.

Mutation rates may be calculated using an in vivo forward mutation assay with, for example, drug resistance as a indicator. With this assay, mutants, i.e. drug resistance, may appear when a relevant gene has a function deficiency or mutation. Alternatively, the mutation rate may be calculated using an in vivo reversion assay. With the reversion assay, a mutant may appear when a relevant gene has recovered function caused by a mutation in the nucleotide sequence. The forward mutation assay may have a higher sensitivity than the reversion assay.

In carrying out the methods of the present invention, introducing the one or more mutator genes can be done using conventional techniques. The one or more mutator genes described herein may be transiently or stably introduced into the target organism using any suitable technique for introduction and expression of an exogenous gene into an organism, such as well established methods of transformation, transduction, and transfection. Such nucleic acid molecule introduction techniques are readily accessible to the skilled artisan and are described in, for example, Ausubel, F. A. et al., editors, (1988), Current Protocols in Molecular Biology, Wiley, New York, N.Y.; Sambrook, J. et al. (1987), Molecular Cloning: A Laboratory Manual, 2nd Ed., and its 3rd Ed. (2001), Cold Spring Harbor Laboratory Press, Cold Spring Harbor, N.Y.; Special issue, Jikken Igaku (Experimental Medicine) "Experimental Method for Gene introduction & Expression Analysis", Yodo-sha, (1997). Moreover, introduction of the one or more mutator genes can be confirmed by equally well established methods, such as by Northern blotting analysis, Western blotting analysis, or other well-known, common techniques. Also described in the literature cited herein are techniques for differentiating cells to produce transformed plants, and methods for obtaining seeds from such transformed plants.

A mutator gene may be expressed in a host organism with a transient mutator gene expression system. With this system, the mutator gene is expressed from a mutator vector without being incorporated into the genome of the organism As part of the transient mutator gene expression system, a mutator gene, or mutator gene fragment, may include a promoter sequence. Different promoters may be used including a wild-type promoter, a constitutive expression promoter, an inducible promoter, or other promoters known by those of skill in the art. In a particular embodiment, the promoter may be a GAL1 inducible promoter controlled by the presence or absence of galactose.

The choice of promoter may affect the level of expression of the mutator gene from the expression vector. A weak or a strong promoter may be combined with a moderate mutator gene to give a weaker or stronger mutator phenotype. A weak promoter may be combined with a strong or a weak mutator gene.

The mutator vector may also include a selection marker along with a mutator gene in order to select those organisms that have been successfully transformed with the mutator vector. The selection marker may be an antibiotic resistance gene, such as ampicillin resistance gene or a Geneticin resistance gene. The selection marker may be an auxotrophic marker gene, such as, for example, URA3 or LEU2.

The mutator vector may include an origin of replication adding to the stability of the mutator vector in the host organism. The origin of replication may be derived from the host organism, a closely related species, or other suitable source. In one particular example, a yeast cell may be transformed with a mutator vector with the ARS1 low-copy, or the 2um ori high-copy origin of replication sequences. In another such example, the mutator vector may comprise the ColE1 moderate-copy or the pUC high-copy origin of replication sequences. The mutator vector may also include a centromere sequence to help maintain the vector in the host organism population during cell division.

The selection of a mutator vector and its particular characteristics, such as a desired promoter, origin of replication, selection marker, etc., may depend on the method of transformation of the host cell, the ploidy of the host cell, the genome size of the host cell, and/or the DNA replication and repair mechanism used by the host cell. In this way, the mutator vector may be customized to best support the desired mutation rate in the host organism.

Mutated organisms may be grown without a specific selective condition. Without a specific selective condition, the organism with the mutator gene accumulates genetic variation because of an accelerated mutation rate, and the accelerated mutation rate leads to an organism with a desired trait. The mutated organisms are then screened for a desired trait, and the organism(s) that demonstrate the desired trait are selected. In one such example, an organism transformed with a mutator gene is grown without a selective condition and then selected for an ability to grow more efficiently or to better produce a desired product or a new product.

The screening and selection of mutated organisms with a desired trait may be accomplished by various well known methods. Target organisms with one or more mutator genes may be exposed to selective conditions that may be used to screen and isolate the organisms with the desired trait. The selective conditions may be chosen from, for example, desired physical growth conditions, the presence of certain chemicals, particular biological conditions, or combinations thereof. The screening of organisms using selective conditions may be used independently or in combination with other screening methods.

The desired physical growth conditions may include a particular pH or a desired range of pH values. In another example , the physical growth conditions may include specific temperatures or temperature ranges, desired atmospheric pressures, or other physical conditions and combinations thereof that are experienced during growth of the organism.

Screening and selection of an organism may be carried out under the presence or absence of chemical substances. The selective chemical conditions may be one or more nutrients or other selective growth medium and/or the presence or absence of hormones, growth factors, organic solvents, antibiotics, halogenated compounds, aromatic compounds, herbicides, analogues, or other suitable chemical conditions.

Mutated organisms with a desired biological trait may be screened by exposing the organisms to selective biological conditions such as a high population density of the organism or exposing the organism to the presence of other species or types of organisms. Organisms can be selected according to their ability to tolerate high population densities or their ability to produce a desired product, such as a pharmaceutical compound. Organisms that survive under these selective biological conditions may do so because they are able to successfully compete for limited resources and/or because they grow synergistically or cooperatively with the surrounding organisms. Those mutated organisms that demonstrate the desired trait may be selected and isolated for further study and/or use.

The introduction of one or more mutator genes may also be accompanied by additional mutations or genetic modifications of the genome of the target organism. For example, the target organism may be transformed with a mutator gene and also be genetically modified at a desired location on the genome of the target organism. The genetic modification may include knockout mutations, point mutations, missense mutations, silent mutations, frame shift mutations, nonsense mutations, insertions or deletions of nucleotides, loss-of-function mutations, gain-of-function mutations, and dominant negative mutations. The genetically modified organisms may be screened for desired traits including more efficient growth and the production of a desired product or compound.

Where a vector is used to introduce one or more mutator genes into the target organism, the vector may be constructed using standard techniques, materials and tools available to the skilled artisan. Any suitable method for introduction of the constructed vector may be used to introduce the one or more mutator genes into the organism. For example, a calcium phosphate method, a DEAE dextran method, an electroporation method, a particle gun (gene gun) method, a calcium chloride method, an electroporation method (Methods. Enzymol., 194, 182 (1990)), a lipofection method, a spheroplast method (Proc. Natl. Acad. Sci. USA, 84, 1929 (1978)), or a lithium acetate method (J. Bacteriol., 153, 163 (1983)) may be used.

In addition, plant expression vectors may be introduced into plant cells using methods well known in the art, such as by a method using an Agrobacterium and a direct inserting method. Examples of methods using Agrobacterium are described in, for example, Nagel et al. (1990). Microbiol. Lett., 67, 325). In such a method, an expression vector suitable for plants is inserted into Agrobacterium by electroporation and the transformed Agrobacterium is introduced into plant cells by a method described in, for example, Gelvin at al., eds, (1994), Plant Molecular Biology Manual (Kluwer Academic Press Publishers)). A mutator gene may be introduced into a plant with a pBL121 vector containing the Cauliflower Mosaic Virus 35S (CaMV35S) promoter. Examples of methods for introducing a plant expression vector directly into plant cells include electroporation (Shimamoto et al. (1989), Nature, 338: 274-276; and Rhodes et al. (1989), Science, 240: 204-207), a particle gun method (Christou et al. (1991), Bio/Technology 9: 957-962), and a polyethylene glycol method (PEG) method (Datta et al. (1990), Bio/Technology 8: 736-740). These methods are well known in the art, and among them, a method suitable for a plant to be transformed may be appropriately selected.

Appropriate vectors, according to those well known in the art, may be chosen according to the desired host and method of replication or integration. For example, a self-replicating vector may be used in a desired yeast strain. In another example, an integration type vector may be used in organisms with unknown origins of replication.

An integration type vector may be used for site-specific homologous recombination. An integration type vector may be used for non-specific random integration. A vector including a loxP sequence may be used in order to facilitate the excision of recombinant DNA sequences from the host organism.

The mutation rate of an organism may be moderated by choosing one or more combinations of vector types. One or more vectors and promoters may be selected depending on the host cell organism and growth conditions. In another example, a low copy plasmid vector may be used. Low copy plasmids may have a more stable phenotype and an efficient curing rate as compared to high copy plasmids. Therefore, it may be more preferable to select low copy plasmids whenever possible. Promoters of different strengths may be selected in order to soften or magnify the strength of mutator phenotype depending on the host cell organism and the growth conditions.

Introduction of the one or more mutator genes as described herein does not involve substitution of one or more of an organism's endogenous DNA polymerase genes with a mutated copy. Even where introduction of the one or more mutator genes involves incorporation of one or more mutator genes into an organism's genome, the introduction is not targeted to replace or affect any of the organism's endogenous genes expressing a DNA polymerase. Nevertheless, as is supported by the experimental examples provided herein, significantly increased mutation rates that facilitate directed evolution of target organisms are achieved through the introduction of one or more mutator genes as described herein. The methods detailed herein provide methods for directed evolution that do not require alteration of an organism's endogenous genes encoding DNA polymerase enzymes. As a consequence, the methods described herein not only allow for the directed evolution and selection of an organism having one or more desired traits, but also facilitate restoration of the organism back to a wild-type mutation rate.

When a modified mutation rate in the evolved organism is no longer desired, a wild-type mutation rate may be restored. Restoring a wild-type mutation rate can be accomplished by any process whereby the one or more mutator genes is (are) removed from the organism, inactivated, or disabled. For example, well established processes for curing an organism of a resident plasmid comprising a mutator gene or by excising or otherwise removing a mutator gene from the host genome may be used to restore a wild-type mutation rate. Alternatively, the vector used to introduce the one or more mutator genes may be constructed such that expression of the one or more mutator genes is inducible under specific conditions. In such an instance, restoration of the wild-type mutation rate can be achieved by subjecting the organism to conditions that do not result in transcription or translation of mutator gene product.

The methods described herein are applicable to a variety of organisms, including, for example, eukaryotic multicellular and unicellular organisms, plants, and prokaryotic organisms. Specific examples of eukaryotic cells to which the methods provided herein may be applied include, but are not limited to mouse myeloma cells, rat myeloma cells, mouse hybridoma cells, Chinese hamster ovary (CHO) cells, baby hamster kidney (BHK) cells, African green monkey kidney cells, human leukemia cells, human colon cancer cells, tobacco plant cells, fungi, and other eukaryotic cells. Additional examples of eukaryotic cells and cell lines include Human Embryonic Kidney cell line HEK293, HeLa cell line, human lymphocytes including B cells and T cells, human hybridoma cells, human myeloma cells, induced pluripotent stem (iPS) cells, embryonic stem (ES) Alternatively, insect cells may be used, such as Sf9 (Spodoptera frugiperda) cells, and S2 (D. melanogaster) cells.

Strain designations for the cells used in the following examples, such as SYD62D-1A, are a reference to the GenBank accession number.

### Examples

### Comparative Example 1

### Generation of Saccharomyces cerevisiae (S. cerevisiae) pol3 mutator vectors

The POL3 gene in S. cerevisiae encodes the catalytic subunit of the yeast DNA polymerase delta which contains two functional domains, 3' to 5' exonuclease and 5' to 3' polymerase domains (Boulet et al.,"Structure and function of Saccharomyces cerevisiae CDC2 gene encoding the large subunit of DNA polymerase III", EMBO J., 1989 Jun; 8(6): 1849-54.). These exonuclease and polymerase activities are required for chromosome replication, repair, and recombination. Furthermore, the 3' to 5' exonuclease of POL3 is involved in mismatch repair and Okazaki fragment maturation as well as proofreading during DNA synthesis (Jin et al., "Multiple Biological Roles of 3' to 5' Exonuclease of Saccharomyces cerevisiae DNA polymerase delta Require Switching between the Polymerase and Exonuclease Domains", Mol. Cell. Biol. 2005 Jan., 25(1): 461-471). herein).

In previous studies, mutations at the predicted active sites of exonuclease motifs EXO I, EXO II, and EXO III were generated by site-directed mutagenesis, and introduced into S. cerevisiae strains. The mutant strains with D321A (aspartic acid to alanine change at amino acid 321) or E323A (glutamic acid to alanine change at amino acid 323) amino acid substitutions without wild-type POL3 showed approximately 370-fold higher mutation rate than wild-type strain. On the other hand, an exonuclease-deficient mutant with wild-type POL3 showed only 5-fold higher mutation rate than wild-type strain. These results suggest that the wild-type POL3 functions in a dominant manner and limits mutations during DNA replication and repair (Simon et al., "The 3' to 5' exonuclease activity located in the POL3 is required for accurate replication", EMBO J., 1991, 10(8): 2165-70, Morrison et al., "Pathway correcting DNA replication errors in Saccharomyces cerevisiae" EMBO J. 1993 Apr; 12(4): 1467-73, Murphy et al., "A method to select for mutator DNA polymerase deltas in Saccharomyces cerevisiae", Genome, 2006, 49: 403-410).

It is believed that the D321A and E323A mutations contribute to a defect in the metal binding ability of the exonuclease function. Also of note, the D321A and E323A mutations in yeast correspond to D316A and E318A amino acid substitutions in human Pol delta (Shevelev, IV and U. Hubscher, "The 3'-5' exonucleases", Nat. Rev. Mol. Cell Biol., 2002 May; 3(5): 364-76).

Another pol3 mutant yeast strain described in previous studies, pol3-L612M, includes a leucine to methionine change at amino acid 612 and is believed to affect the DNA polymerase fidelity (Li et al., "Sensitivity to Phosphonoacetic Acid: A New Phenotype to Probe DNA Polymerase delta in Saccharomyces cerevisiae", Genetics, 2005 Jun.; 170: 569-580, Venkatesan et al., "Mutator phenotypes caused by substitution at a conserved motif A residue in eukaryotic DNA polymerase delta", J. Biol. Chem., 2006 Feb. 17; 281(7): 4486-94). In the experiments of Li et al. and Venkatesan et al., the pol3-L612M mutant strain showed mutation rates that were approximately 1.6 to 7.0-fold higher relative to a wild-type yeast strain.

The pol3-L612M mutant strain in the previous studies was constructed by integrating the pol3-L612M allele into the chromosomal POL3 gene by targeted integration or by plasmid-shuffling method, thereby disrupting the endogenous POL3 gene. In order to restore the mutant strains to a wild-type mutation rate, the endogenous wild-type POL3 would need to be restored by another oligonucleotide-mediated mutagenesis. The use of mutator plasmids allows the continued expression of the endogenous wild-type POL3 and provides for an efficient restoration of a wild-type mutation rate by curing the yeast strains from the mutator plasmid. In this way, the increased mutation rate of an organism is stopped when a desired trait is acquired, preventing the unwanted mutation of other traits.

### A. Construction of YCplac111 mutator plasmid vectors

The DNA fragments of the wild-type genomic POL3 gene (SEQ ID NO: 1) including coding (3291 bp) and non-coding sequence (1000 bp upstream and 500 bp downstream) were cloned from S. cerevisiae genomic DNA by PCR method using primers, ScPOL3pro-S: 5'-AGTCGAGTCGACTGTTTTCCTTGATGGCACGGT (SEQ ID NO: 2), and ScPOL3ter-AS:
5'-AGTCGAGAATTCTGGAGTGCTGGTGTCATATTA (SEQ ID NO: 3). The amplified DNA fragments were inserted into the multi-cloning sites (SalI and EcoRI) of YCplac111 to construct YCplac111/POL3 plasmid vector. For the exonuclease-deficient pol3 (pol3-01) plasmid vector, a D321A substitution (aspartic acid to alanine amino acid change at amino acit 321) and a E323A substitution (glutamic acid to alanine substitution at amino acid 323) were produced in the YCplac33/POL3 plasmid using the QuikChange Kit (Stratagene) with a primer, Scpo13-01:
5'-GCGTATCATGTCCTTTGCTATCGCGTGTGCTGGTAGGATTG (SEQ ID NO: 4), resulting in YCplac111/pol3-01 plasmid vector.

For construction of YCplac111/pol3-01+L612X plasmid vectors, a leucine at amino acid 612 was changed to a desired amino acid, as shown in Table 1, and was produced in the YCplac1 11/po13-01 plasmid by using the QuikChange Kit (Stratagene), and the following primers:
Scpol3L612A:
   5'-GCAACTTTGGATTTCAATTCTGCTTATCCAAGTATTATGATGGCG (SEQ ID NO: 5),
Scpol3L612C:
   5'-GCAACTTTGGATTTCAATTCTTGTTATCCAAGTATTATGATGGCG (SEQ ID NO: 6),
Scpol3L612D:
   5'-GCAACTTTGGATTTCAATTCTGATTATCCAAGTATTATGATGGCG (SEQ ID NO: 7),
Scpol3L612E:
   5'-GCAACTTTGGATTTCAATTCTGAGTATCCAAGTATTATGATGGCG (SEQ ID NO: 8),
Scpo13L612F:
   5'-GCAACTTTGGATTTCAATTCTTTCTATCCAAGTATTATGATGGCG (SEQ ID NO: 9),
Scpol3L612G:
   5'-GCAACTTTGGATTTCAATTCTGGATATCCAAGTATTATGATGGCG (SEQ ID NO: 10),
Scpol3L612H:
   5'-GCAACTTTGGATTTCAATTCTCATTATCCAAGTATTATGATGGCG (SEQ ID NO: 11),
Scpol3L6121:
   5'-GCAACTTTGGATTTCAATTCTATATATCCAAGTATTATGATGGCG (SEQ ID NO: 12),
Scpol3L612K:
   5'-GCAACTTTGGATTTCAATTCTAAGTATCCAAGTATTATGATGGCG (SEQ ID NO: 13),
Scpol3L612M:
   5'-GCAACTTTGGATTTCAATTCTATGTATCCAAGTATTATGATGGCG (SEQ ID NO: 14),
Scpol3L612N:
   5'-GCAACTTTGGATTTCAATTCTAATTATCCAAGTATTATGATGGCG (SEQ ID NO: 15),
Scpol3L612P:
   5'-GCAACTTTGGATTTCAATTCTCCATATCCAAGTATTATGATGGCG (SEQ ID NO: 16),
Scpol3L612Q:
   5'-GCAACTTTGGATTTCAATTCTCAGTATCCAAGTATTATGATGGCG (SEQ ID NO: 17),
Scpol3L612R:
   5'-GCAACTTTGGATTTCAATTCTCGTTATCCAAGTATTATGATGGCG (SEQ ID NO: 18),
Scpol3L612S:
   5'-GCAACTTTGGATTTCAATTCTTCTTATCCAAGTATTATGATGGCG (SEQ ID NO: 19),
Scpol3L612T:
   5'-GCAACTTTGGATTTCAATTCTACTTATCCAAGTATTATGATGGCG (SEQ ID NO: 20),
Scpol3L612V:
   5'-GCAACTTTGGATTTCAATTCTGTTTATCCAAGTATTATGATGGCG (SEQ ID NO: 21),
Scpol3L612W:
   5'-GCAACTTTGGATTTCAATTCTTGGTATCCAAGTATTATGATGGCG (SEQ ID NO: 22),
Scpol3L612Y:
   5'-GCAACTTTGGATTTCAATTCTTACTATCCAAGTATTATGATGGCG (SEQ ID NO: 23).

### B. Construction of YCplac33 mutator plasmid vectors

Each of the YCplac1 11/pol3-01 or YCplac111/pol3-01+L612M plasmids was digested with two restriction enzymes, SalI and EcoRI. The DNA fragments of the genomic pol3 mutant genes were purified, and then inserted into the multi-cloning sites (SalI and EcoRI) of the YCplac33 plasmid to construct YCplac33/po13-01 and YCplac33/po13-01+L612M plasmid vectors.

### C. Construction of YEplac195 mutator plasmid vectors

Each of the YCplac111/pol3-01 or the YCplac111/pol3-01+L612M plasmids was digested with two restriction enzymes, SalI and EcoRI. The DNA fragments of the genomic pol3 mutant genes were purified, and then inserted into the multi-cloning sites (SalI and EcoRI) of the YEp1ac195 plasmid to construct YEp1ac195/po13-01 and YEplac195/pol3-01+L612M plasmid vectors.

### D. Construction of YIplac33 mutator vectors

The YCplac33 vector was digested with two restriction enzymes, SpeI and NheI, and then the DNA fragments were treated with T4 DNA polymerase to generate blunt-ends on the DNA fragments. The blunt-ended DNA fragments were ligated with T4 DNA ligase to generate the YIp1ac33 vector. Each of the YCplac1 11/po13-01 or the YCplac1 11/pol3-01+L612M plasmids was digested with two restriction enzymes, SalI and EcoRI. The DNA fragments of the genomic pol3 mutant genes were purified and then inserted into the multi-cloning sites (SalI and the EcoRI) of the YIp1ac33 plasmid to construct the YIplac33/pol3-01 and the YIplac33/pol3-01+L612M vectors.

### E. Construction of YCplac33/Gal1p galactose inducible mutator plasmid vector

GAL1 promoter region (450 bp) was cloned from S. cerevisiae genomic DNA by PCR using the primers, GAL1U1: 5'-ATACTGCAAGCTTACGGATTAGAAGCCGCCGAG (SEQ ID NO: 24) and GAL1D1: 5'-CCCTATCTGCAGGGGGTTTTTTCTCCTTGACG (SEQ ID NO: 25). The amplified DNA fragments were digested with two restriction enzymes, HindIII and PstI, and then inserted into the multi-cloning sites (HindIII and PstI) of YCplac33 plasmid to construct YCplac33/Gal1p plasmid vector. The DNA fragments of the pol3-01+L612M coding sequence were amplified from YCplac111/po13-01+L612M plasmids by PCR using the primers, SCPDAESAL1: 5'-ATACGCGTCGACATGAGTGAAAAAAGATCCCTTC (SEQ ID NO: 26) and SCPDAISAC1: 5'-GCCACCGAGCTCGAAGGAACAGATCTTACAAGC (SEQ ID NO: 27). The amplified DNA fragments were digested with two restriction enzymes, SalI and SacI, and then inserted into the multi-cloning sites (SalI and SacI) of the YCplac33/Gal1p plasmid to construct YCpla33/Gal1p/pol3-01+L612M plasmid vectors.

### Comparative Example 2

### Transformation of S. cerevisiae to generate mutator strains

Two yeast strains were used in this example; a haploid strain, SYD62D-1A (MATalpha, ura3-52, his3-delta300, trpl- delta90, leu2-3, 112, lys2-801, ade2-2) and a diploid strain, YPH501 (MATa/alpha ura3-52/ura3-52 lys2-801/lys2-801 ade2-101/ade2-101 trp1-delta63/trp1-delta63 his3-delta200/his3-delta200 leu2-delta1/leu2-delta1). Each of these yeast strains was grown in YPD medium (1% yeast extract, 2% polypepton, 2% glucose) and the competent cells were prepared by using Frozen EZ Yeast Transformation II (ZymoResearch).

### A. YCplac111 mutator strains

Each of the YCplacl 1 1/pol3-01 or YCplac111/pol3-01+L612X mutator plasmid vectors (200ng DNA) was introduced into 10ul of SYD62D-1A competent cells by using Frozen EZ Yeast Transformation II (ZymoResearch). The cells were spread onto synthetic complete medium, omitted leucine, agar plates (SC/-Leu: 0.67% Yeast Nitrogen Base w/o amino acids, 0.69g/L CSM-Leu (FORMEDIUM™), 2% Glucose, 2% agar), and incubated at 30 degree Celsius for three days. Each of the transformants was isolated and designated as YCplac1 1 1/pol3-01 or YCplacl111pol3-01+L612X mutator strains, where X represents a desired amino acid residue. The endogenous wild-type POL3 gene of the transformants remained intact and active.

### B. YCplac33 mutator strains

Each of the YCplac33/pol3-01 or YCplac33/pol3-01+L612M mutator plasmid vectors (200ng DNA) was introduced into the 10ul of SYD62D-1A competent cells by using Frozen EZ Yeast Transformation II (ZymoResearch). The cells were spread onto synthetic complete medium, omitted uracil, agar plates (SC/-Ura: 0.67% Yeast Nitrogen Base w/o amino acids, 0.77g/L CSM-Ura (FORMEDIUM™), 2% Glucose, 2% agar), and incubated at 30 degree Celsius for three days. Each of the transformants was isolated and designated as YCplac33/pol3-01 or YCplac33/pol3-01+L612M mutator strains. The endogenous wild-type POL3 gene of the transformants remained intact and active.

### C. YEplac195 mutator strains

Each of the YEplac195/pol3-01 or YEp1ac195/po13-01+L612M mutator plasmid vectors (200ng DNA) was introduced into the 10ul of SYD62D-1A competent cells by using Frozen EZ Yeast Transformation II (ZymoResearch). The cells were spread onto SC/-Ura, and incubated at 30 degree Celsius for three days. Each of the transformants was isolated and designated as YEp1ac195/po13-01 or YEplac195/pol3-01+L612M mutator strains. The endogenous wild-type POL3 gene of the transformants remained intact and active.

### D. YIp1ac33 mutator strains

Each of the YIplac195/pol3-01 or YEp1ac195/po13-01+L612M mutator plasmid vectors was digested with a restriction enzyme, KpnI, to linearize the vectors. Each of the linearized vectors (1000ng) was introduced into the 100ul of SYD62D-1A competent cells by using Frozen EZ Yeast Transformation II (ZymoResearch). The cells were spread onto SC/-Ura, and incubated at 30 degree Celsius for three days. Each of the transformants was isolated and genotyped by PCR using primers, M13YCp: 5'-ACGTTGTAAAACGACGGCCAG (SEQ ID NO: 28) and ScPOL3IC1: 5'-TTTACGGTGACACTGATTCCG (SEQ ID NO: 29), to confirm that the mutator vector was integrated into the POL3 locus. Each of the positive transformants was designated as YIplac33/pol3-01 or YIplac33/pol3-01+L612M mutator strains. The endogenous wild-type POL3 gene of the transformants remained intact and active.

### E. YCplac33/Gal1p mutator strains

YCplac33/Gal1p/pol3-01 or YCplac33/Gal1p/pol3-01+L612M mutator plasmid vectors (200ng DNA) were introduced into the 10ul of YPH501 competent cells by using Frozen EZ Yeast Transformation II (ZymoResearch). The cells were spread onto SC/-Ura, and incubated at 30 degree Celsius for three days. Each of the transformants was isolated and designated as YCplac33/Gal1p/pol3-01+L612M mutator strains. To express mutator pol3 genes, the transformants were transferred to SC/-Ura/Galactose (in place of 2% Glucose to 2% Galactose) agar plates and cultured at 30 degree Celsius for five to seven days. The endogenous wild-type POL3 gene of the transformants remained intact and active.

### Fluctuation Analysis of the Mutation Rate at the CAN1 locus in S. cerevisiae

A CAN1 forward mutation assay designed to detect the canavanine sensitive (Can^{s}) to canavanine resistant (Can^{R}) forward mutation was performed on five independent colonies from each plate.

The experimental method to study mutation rates, i.e., the probability of a mutation per cell per division (or generation), is referred to as fluctuation analysis. There are various mathematical methods known by those of skill in the art for estimating the mutation rate from the number of mutations per culture in a fluctuation analysis. These mathematical methods involve using the observed distribution in a number of parallel cultures to estimate the probable number of mutations per culture and then using the probable number of mutations to calculate the mutation rate.

For this example, the mutation rate (MR) at the CAN1 locus is equal to the mutation coefficient (m) divided by the total number of cells in the culture (N), i.e. MR=m/N. The mutation coefficient may be defined as m=r0/(r0/m), where r0 is the number of Can^{R} resistant colonies for each clone. The value of the quantity r0/m is found using the Lea-Coulson's Index as described in Lea and Coulson, "The distribution of the number of mutants in bacterial populations" J. Genet. 1948, vol. 49, 264-285.

The CAN1 forward mutation assay was repeated three times for each of the plates and the average mutation rates were determined. The results are shown in Table 1, 2 and 3.
[Table 1]

**TABLE 1**

| **Mutation rates (MR) at the CAN1 locus in *S. cerevisiae* (SYD62D-1 A, a haploid strain)** | | | | | |
|---|---|---|---|---|---|
| ***Pol3* vectors** | **1** | **2** | **3** | **Ave.** | **Relative MR** |
| **No vector** | **2.3×10⁻⁷** | **2.5×10⁻⁷** | **2.3×10⁻⁷** | **2.4×10⁻⁷** | **1** |
| **YCplac111/*pol3-01*** | **1.4×10⁻⁶** | **2.5×10⁻⁶** | **8.1×10⁻⁷** | **1.5×10⁻⁶** | **6.3** |
| **/*pol3-01+L612A*** | **5.9×10⁻⁶** | **2.6×10⁻⁵** | **8.8×10⁻⁶** | **1.4×10⁻⁵** | **58** |
| ***lpol3-01+L612C*** | **lethal** | **lethal** | **lethal** | **-** | **-** |
| **/*pol3-01+L612D*** | **1.4×10⁻⁶** | **2.9×10⁻⁶** | **3.8×10⁻⁶** | **2.7×10⁻⁶** | **11** |
| **/*pol3-01+L612E*** | **7.2×10⁻⁶** | **1.0×10⁻⁵** | **8.2×10⁻⁶** | **8.4×10⁻⁶** | **35** |
| **/*pol3-01+L612F*** | **lethal** | **lethal** | **lethal** | **-** | **-** |
| **/*pol3-01+L612G*** | **2.0×10⁻⁵** | **2.3×10⁻⁵** | **4.7×10⁻⁵** | **3.0×10⁻⁵** | **130** |
| **/*pol3-01+L612M*** | **3.7×10⁻⁵** | **3.0×10⁻⁵** | **2.5×10⁻⁵** | **3.1×10⁻⁵** | **130** |
| **/*pol3-01+L612N*** | **4.2×10⁻⁶** | **5.0×10⁻⁶** | **9.5×10⁻⁶** | **6.2×10⁻⁶** | **26** |
| **/*pol3-01+L612Q*** | **1.5×10⁻⁵** | **1.5×10⁻⁴** | **4.5×10⁻⁶** | **5.6×10⁻⁵** | **230** |
| **/*pol3-01+L612S*** | **1.9×10⁻⁵** | **1.8×10⁻⁵** | **4.8×10⁻⁵** | **2.9×10⁻⁵** | **120** |
| **/*pol3-01+L612V*** | **7.8×10⁻⁶** | **9.5×10⁻⁶** | **4.9×10⁻⁶** | **7.4×10⁻⁶** | **31** |
| **/*pol3-01+L612W*** | **3.6×10⁻⁵** | **3.7×10⁻⁵** | **2.4×10⁻⁵** | **3.2×10⁻⁵** | **130** |
| **/*pol3-01+L612Y*** | **lethal** | **lethal** | **lethal** | **-** | **-** |

| | | | | | |
|---|---|---|---|---|---|
| *** Mutation rates were determined by fluctuation tests.** | | | | | |

[Table 2]

**TABLE 2**

| **Mutation rates (MR) at the CAN1 locus in *S. cerevisiae* (SYD62D-1A, a haploid strain)** | | | | | |
|---|---|---|---|---|---|
| ***Pol3* vectors** | **1** | **2** | **3** | **Ave.** | **Relative MR** |
| **No vector** | **2.3×10⁻⁷** | **2.5×10⁻⁷** | **2.3×10⁻⁷** | **2.4×10⁻⁷** | **1** |
| **YCpIac33/*pol3-01*** | **1.1×10⁻⁶** | **1.2×10⁻⁶** | **5.7×10⁻⁷** | **9.6×10⁻⁷** | **4.0** |
| **YCpIac33/*pol3-01+L612M*** | **3.4×10⁻⁵** | **9.9×10⁻⁶** | **8.0×10⁻⁶** | **1.7×10⁻⁵** | **71** |
| **YEpIac195/*pol3-01*** | **4.4×10⁻⁶** | **2.9×10⁻⁶** | **1.1×10⁻⁶** | **2.8×10⁻⁶** | **12** |
| **YEpIac195/*pol3-01+L612M*** | **9.9×10⁻⁶** | **1.4×10⁻⁵** | **5.9×10⁻⁵** | **2.8×10⁻⁵** | **120** |
| **YIplac33/*pol3-01*** | **6.1×10⁻⁷** | **1.2×10⁻⁶** | **1.3×10⁻⁶** | **1.0×10⁻⁶** | **4.2** |
| **YIplac33/*pol3-01+L612M*** | **1.0×10⁻⁵** | **7.7×10⁻⁵** | **7.1×10⁻⁵** | **5.3×10⁻⁵** | **220** |

| | | | | | |
|---|---|---|---|---|---|
| *** Mutation rates were determined by fluctuation tests.** | | | | | |

[Table 3]

**TABLE 3**

| **Mutation rates (MR) at the CAN1 locus in *S*. *cerevisiae* (YPH501, a diploid strain)** | | | | | |
|---|---|---|---|---|---|
| ***Pol3* vectors** | **1** | **2** | **3** | **Ave.** | **Relative MR** |
| **No vector** | **4.6×10⁻⁸** | **5.4×10⁻⁸** | **5.6×10⁻⁸** | **5.2×10⁻⁸** | **1** |
| **YCplac33/GAL1p/*pol3-01+L612M*** | **1.7×10⁻⁵** | **3.2×10⁻⁵** | **3.2×10⁻⁶** | **1.7×10⁻⁵** | **330** |

| | | | | | |
|---|---|---|---|---|---|
| *** Mutation rates were determined by fluctuation tests.** | | | | | |

With reference to TABLE 1, the wild-type mutation rate, i.e. the CAN1 mutation rate of a haploid yeast strain (SYD62D-1 A) without a mutator plasmid vector, was approximately 2.4x10⁻⁷. The mutation rate at the CAN1 locus of the YCplac111/pol3-01 mutator strain averaged approximately 1.5x10⁻⁶, only 6.3 times greater than wild-type. On the other hand, the subset of pol3-01+L612X mutator strains L612A, L612G, L612M, L612Q, L612V, and L612W, exhibited increased mutation rates ranging from 58 times to 230 greater than that of the wild-type strain. The pol3-01+L612X mutator strains L612C, L612F, and L612Y, showed a lethal phenotype. Mutator plasmid vectors YCplac111/pol3-01+L612C, L612F, and L612Y, were introduced into a diploid yeast strain (YPH501) to confirm whether the mutant strains were viable or not. The results showed that YCplac111/pol3-01+L612C, L612F, and L612Y diploid strains were viable (data not shown), suggesting that the mutants appear to have stronger mutator phenotype than that of YCplac111/pol3-01+L612Q. Moreover, the subset of mutant strains pol3-01+L612H, L612I, L612K, L612R, and L612T, showed similar or less mutation rates relative to the pol3-01 single mutant. These results indicate that YCplac111/pol3-01+L612X mutator vectors can confer various mutation rates ranging from 2 times to over 230 times relative to wild-type strains.

Therefore, the combination of the pol3-01 and a subset of L612X (A, G, M, Q, V, and W) mutants (TABLE 1), in a single mutator vector yield a mutation rate that was synergistically higher than the mutation rate of that of the pol3-01 or the L612X (A, G, M, Q, V, and W) mutations individually. Furthermore, the pol3-01+L612X (A, G, M, Q, V, and W) genotype on the mutator plasmids appears to act in a dominant-negative fashion over the wild-type POL3 expression. That is, while the wild-type POL3 is still intact and expressed, the pol3 mutants expressed from the mutator vectors are likely competitive inhibitors of the endogenous DNA polymerase delta, thereby decreasing the overall DNA replication fidelity.

With reference to TABLE 2, various types of mutator vectors, such as YCplac33, YEplac195, and YIplac33, can confer strong mutator phenotype to haploid or diploid yeast strains. These vectors contain the URA3 selection marker, that is useful to isolate cells after curing them of the mutator vectors (described in detail in Example 3). The wild-type mutation rate, i.e. the CAN1 mutation rate of a SYD62D-1A yeast clone without a mutator plasmid vector, was approximately 2.4x10⁻⁷. The mutation rate at the CAN1 locus of the pol3-01 strain averaged approximately 9.6x10⁻⁷, only 4.0 times greater than wild-type. However, the fluctuation analysis revealed that the combination of the pol3-01 and the L612M mutations used with the YCplac33 and the YEplac195 plasmid vectors cause an approximate 10 times increase in the mutation rate of the CAN 1 locus relative to the pol3-01 mutation alone. When using the YIp1ac33 vector, the pol3-01+L612M combination shows a mutation rate that is more than 50 times greater than using the pol3-01 mutation alone.

Additionally, when compared to the wild-type mutation rate, the mutation rate for the YCplac33/pol3-01+L612M mutator plasmid was 71 times higher. For the YEp1ac195/po13-01+L612M mutator plasmid, the mutation rate was 120 times that of the wild-type strain. For the YIplac33/pol3-01+L612M mutator plasmid, the mutation rate was 220 times that of the wild-type strain.

As shown in TABLE 3, a galactose inducible mutator vector YCplac33/Gal1p/pol3-01+L612M can confer strong and transient mutator phenotype to yeast strains. The wild-type mutation rate, i.e. the CAN1 mutation rate of the diploid yeast strain YPH501 without a mutator plasmid vector, was approximately 5.2x10⁻⁸. In contrast, the YCplac33/Gal1p/pol3-01+L612M mutator vector in the YPH501 yeast strain showed approximately 330 times greater mutation rate than that of the wild-type strain.

### Comparative Example 3

### Restoration of Wild-Type Mutation Rate in Yeast

When a desired trait has been achieved in the mutated yeast, the mutation rate of the yeast transformed with the YCplac33 and YEp1ac195 type plasmid vectors may be restored back to a wild-type mutation rate. The wild-type mutation rate is restored to genetically fix the desired trait in the mutated yeast and to avoid further mutations in the yeast that may not be desirable. The wild-type mutation rate is restored by curing the transformed yeast cells from the YCplac33 and YEp1ac195 mutator plasmid vectors by culturing the yeast on SC medium containing 1g/L 5-Fluoroorotic acid (5-FOA), a selective culture medium that only allows the growth of cells that do not express any functional URA3 gene.

In yeast cells transformed with a YIp1ac33 integration-type mutator vector, the mutator vector may be excised from the POL3 locus of the yeast genome by homologous recombination, thereby returning the cells to a wild-type mutation rate. To cure the YIplac33 mutator vector from the transformants, at least 107 cells are spread on SC/5-FOA and the surviving yeast colonies are genotyped to confirm restoration of the wild-type POL3.

### Example4

### Generation of Pold1 Mutant Expression Vector

The catalytic subunit of DNA polymerase delta is encoded by the Pold1 gene. In this example, a Chinese Hamster Ovary (CHO) cell cDNA library was made, and the Pold1 cDNA was isolated (SEQ ID NO: 87). A D398A (aspartic acid to alanine change at amino acid 398) mutation was introduced into the Pold1 3' to 5' exonuclease active site and a L602M (leucine to methionine change at amino acid 602) mutation was introduced into the Pold1 polymerase domain (Goldsby et al., "Defective DNA polymerase delta proofreading causes cancer susceptibility in mice", Nat Med., 2001 Jun: 7(6), 638-9, Goldsby et al., "High incidence of epithelial cancers in mice deficient for DNA polymerase proofreading", PNAS, November 26, 2002, vol. 99 (24), 15560-15565, Venkatesan et al., "Mutation at the polymerase active site of mouse DNA polymerase delta increases genomic instability and accelerates tumorigenesis", Mol Cell Biol. 2007 Nov;27(21): 7669-82). The mutations were introduced using the oligonucleotides 5'-GGCTATAATATTCAGAACTTTGCCCTCCCATACCTCATCTCG CGC (SEQ ID NO: 30 (alanine anticodon underlined) and 5'-CCCTGGATTTCTCCTCTATGTACCCATCCATCATG (SEQ ID NO: 31) (methionine anticodon underlined), respectively (Quikchange, Stratagene), thereby obtaining a Pold1+D398A mutant and Pold1+D398A+L602M mutant. The D398A mutation corresponds to a D401A amino acid substitution in human Pol delta as discussed in Shevelev, IV and U. Hubscher, "The 3' 5' exonucleases", Nat. Rev. Mol. Cell Biol., 2002 May; 3(5):364-76.

For cloning and expression of the Pold1 mutants, a pCMV-Script (Stratagene) mammalian expression vector was used. The Pold1+D398A mutant and Pold1+D398A+L602M were inserted into the expression vector after the cytomegalovirus (CMV) promoter (G418 resistant) to construct Pold1+D398A and Pold1+D398A+L602M expression vectors. Two loxP sites were inserted into the construct allowing the extraction of the Pold1 mutant from the CHO cell genome with Cre recombinase.

### Example5

### Fluctuation Analysis of Mutation Rates at the Oua^{R}locus in CHO Cells

The Pold1+D398A mutant expression vector and the Pold1+D398A+L602M mutant expression vector were transfected, using standard transfection methods, into CHO-DXB11 cells. After transfection, stable Pold1 mutant expression cell lines, DXB11/Pold1+D398A and DXB11/Pold1+D398A+L602M were obtained. The CHO-DXB 11 (without vector), DXB11/Pold1+D398A, and DXB11/Pold1+D398A+L602M cells were placed in a standard culture medium including 2mM ouabain. In order to eliminate pre-existing ouabain resistant cells, cell populations that grew from 1,000 cells to 5x10⁷ cells were used.

The number of ouabain resistant (Oua^{R}) colonies that appeared after 14 days of culture and the total number of disseminated cells (1∼3x10⁷) were used to determine the mutation rates according to the Lea Coulson's index as discussed previously. The fluctuation analysis was repeated three times for each cell culture, and the average mutation rates were determined. The results of the fluctuation analysis of mutation rates at the Oua^{R} locus in CHO cells are shown in TABLE 4.
[Table 4]

**TABLE 4**

| **Mutation rates (MR) at the Ouabain^{R} (Oua^{R}) locus in CHO cell lines** | | | | | |
|---|---|---|---|---|---|
| **Pold1 vectors** | **1** | **2** | **3** | **Ave.** | **Relative MR** |
| **No vector** | **3.6×10⁻⁸** | **3.6×10⁻⁸** | **3.6×10⁻⁸** | **3.6×10⁻⁸** | **1** |
| ***Pold1+D398A*** | **5.4×10⁻⁸** | **5.4×10⁻⁸** | **6.6×10⁻⁸** | **5.8×10⁻⁸** | **1.6** |
| ***PoId1+D398A+L602M*** | **1.4×10⁻⁶** | **1.4×10⁻⁶** | **1.1×10⁻⁶** | **1.3×10⁻⁶** | **36** |

| | | | | | |
|---|---|---|---|---|---|
| *** Mutation rates were determined by fluctuation tests.** | | | | | |

The wild-type mutation rate at the Oua^{R} locus in CHO cells without a vector averaged approximately 3.6x10⁻⁸. The CHO cells with the Pold1+D398A mutation demonstrated a mutation rate of approximately 5.8x10⁻⁸, 1.6 times greater than the wild-type mutation rate. However, the CHO cell with the Pold1+ D398A+L602M mutation had a mutation rate of approximately 1.3x10⁻⁶, or 36 times the wild-type mutation rate.

### Example6

### Restoration of the Wild-type Mutation Rate in CHO Cells

The region between two copies of loxP site is excised by site-specific recombination catalyzed by Cre-recombinase. In order to restore the wild-type mutation rate, CHO mutator cells are transfected with Cre-recombinase expression vector (puromycin re-sistance). Those CHO cells that are selected in the presence of puromycin, are those cells with a restored wild-type mutation rate. The wild-type mutation rate is restored as the Pold1 mutant is excised from the genome by the Cre-loxP site-specific recombination.

### Comparative Example 7

### Generating Clotrimazole (CTZ) Resistant Yeast

YCplac33/pol3-01+L612M vector was introduced into a S. cerevisiae strain, that was ura3 deficient mutant of Taiken 396 (Taiken 396 ura3-) to create the Taiken 396 mutator yeast strain. The Taiken 396 mutator strain was grown on SD medium (0.67% yeast nitrogen base w/o amino acids, 2% glucose), and cultured with shaking (180rpm) for 24 hours at 30 degree Celsius. The culture was then plated on SD agar medium containing 25mg/L clotrimazole (CTZ) and cultured for three days at 30 degree Celsius. The parent yeast strain Taiken 396 does not normally survive on SD agar medium containing 25mg/L CTZ. Mutated yeast colonies with the desired survival trait, i.e. that survived in the presences of 25mg/L CTZ, were isolated.

To fix the clotrimazole resistance trait in the yeast genome and remove the YCplac33/pol3-01+L612M vector, the isolated yeast clones were plated onto SC agar medium (0.67% yeast nitrogen base w/o amino acids, 0.079% complete supplement mix, 2% glucose, 2% agar) containing 1g/L 5-Fluoroorotic acid and cultured for three days at 30 degree Celsius. The surviving clones were isolated and fixed for the CTZ resistance trait and free of the mutator vector. To rescue the ura3 deficiency of the CTZ resistant strains, the competent cells were transformed with DNA fragments of the intact URA3 gene by homologous recombination and plated onto SD agar medium for three days at 30 degree Celsius. The positive clones were isolated and confirmed CTZ resistance and non-auxotrophy.

### Comparative Example 8

### Fermentation Ability Test

A wild-type strain and the CTZ resistant strain of Taiken 396 were inoculated into YPD medium (1% yeast extract, 2% polypepton, 2% glucose) and cultured with shaking (180 rpm) for 24 hours at 30 degree Celsius.

Each of the cultured cells was collected from the YPD medium and rinsed with distilled water and then inoculated at a final concentration of 2x10⁶ cells/mL into 20 mL of YPS medium (1% yeast extract, 2% polypepton, 20% sucrose). Each of the cells was grown in YPS medium at 30 degree Celsius with stirring twice a day. Ethanol concentration and cell viability of the wild-type strain or CTZ resistant strain in the YPS medium were measured on days three and four after inoculation.

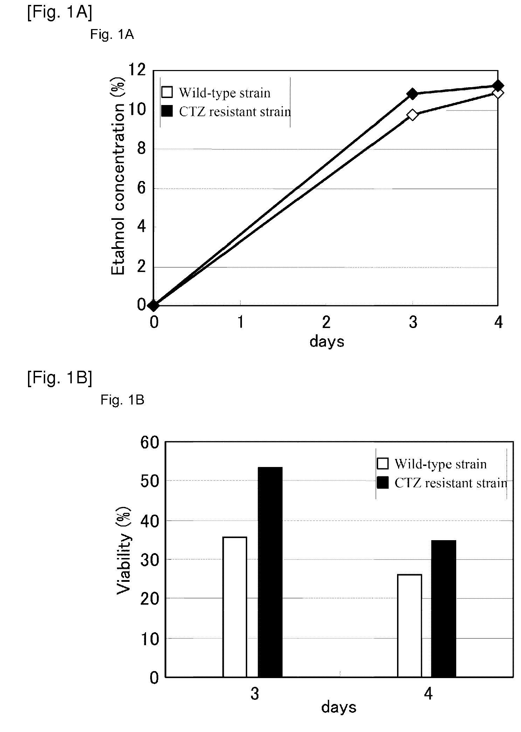

The CTZ resistant strain showed higher ethanol tolerance than that of the wild-type strain after three and four days of the culture (Fig.1A) The CTZ resistant strain also showed approximately 20% higher viability than that of the wild-type strain after 3 days, and approximately 10% higher viability after 4 days (Fig.1B).

### Comparative Example 9

### Conferring Herbicide Resistance to the Tobacco Plant

### A. Isolation of tobacco DNA polymerase delta gene

The tobacco plant Nicotiana tobacum cv. strain Bright Yellow 2 ("tobacco BY-2") was used to explore the ability of a mutator gene to direct the evolution of a plant.

To isolate the catalytic subunit of DNA polymerase delta(Pold1) gene of tobacco BY-2, the Pold gene sequences of Arabidopsis thaliana and Glycine max (Dicotyledons), Oryza sativa (Monocotyledons), and Chlorella vulgaris (Cyanobacteria) were obtained and multiple sequence alignments were made of the pold amino acid sequences. The sequence alignment also included comparison with the amino sequence of POL3 of S. cerevisiae (see, i.e., FIG. 3). After the sequence alignments, highly conserved regions of the pold sequences were identified and used for the design of PCR primers, PBOX1-Fw: 5'-GT(G/T)CA(C/T)GG(A/C/G)TTGA(A/G)CC(A/C)TA(C/T)TT(C/T)TAC (SEQ ID NO: 32) and PBOX9-Rv: 5'-CA(A/G)(A/G)CC(A/C)GC(A/G)TA(C/G/T)C(G/T)CTTCTT (SEQ ID NO: 33) to isolate the tobacco BY-2 Pold gene sequence (SEQ ID NO:88).

The cDNA of the BY-2 cell was prepared by reverse transcription (RT) using ReveTraAce (Toyobo, Osaka, Japan) with oligo(dT) primer, and total RNA that was purified from tobacco BY-2 cell. A partial fragment of BY-2 pold1 cDNA was cloned by RT-PCR using Fusion DNA polymerase (FINZYME™), the primers (PBOX1-Fw and PBOX9-Rv), and the BY-2 RNA as a template. The cDNA sequence was used to construct primers to isolate the entire tobacco BY-2 Pold1 gene using 3'-RACE and 5'-RACE.

B. Modification of 3' to 5' exonuclease region and the polymerase fidelity region The nucleotide sequence of the tobacco BY-2 Pold1 gene was modified to produce one or more mutations in the 3' to 5' exonuclease region and/or the polymerase fidelity region of the tobacco BY-2 DNA polymerase. More particularly, the 3' to 5' exonuclease region of the Pold1 gene was modified to generate exonuclease-deficient mutations comprising a D275A (aspartic acid to alanine change at amino acid 275) and a E277A (glutamic acid to alanine change at amino acid 277) amino acid change, which was designated as Ntpold1^{exo-}. The 3' to 5' exonuclease region and the polymerase fidelity region of the Pold1 gene were modified to create exonuclease-deficient mutations and a low fidelity mutation comprising the D275A and E277A amino acid changes along with a L567D (leucine to aspartic acid change at amino acid 567) amino acid change, which was designated as Ntpold1^{exo-}+L567M. The D275A and E277A mutations in the exonuclease region of tobacco Pold1 correspond to D316A and E318A amino acid substitutions in human Pol delta (Shevelev, IV and U. Hubscher, "The 3' 5' exonucleases", Nat. Rev. Mol. Cell Biol., 2002 May; 3(5):364-76).

### C. Transformation of tobacco cells

The Ntpold1^{exo}- mutant gene was inserted into a pBI121 plant transformation vector to generate a pBI/Ntpold^{exo} mutant vector. The Ntpoldl^{exo}+L567M mutant gene was also inserted into a pBI121 vector to create the pBI/Ntpold1^{exo}+L567M mutant vector. The pBI/Ntpold^{exo-} mutant vector and the pBI/Ntpold1^{exo-}+L567M mutant vector were introduced into Agrobacterium GV3101, and transformation into the tobacco BY-2 culture cells was performed by simultaneous culturing of the Agrobacterium and tobacco BY-2 cells. After simultaneous culturing, transformants were selected in a selective medium containing kanamycin and claforan.

As a control, a pBI121 vector without a Pold1 mutator gene, pBI/empty, which contained no genetic insertion downstream of the CaMV35S promoter of pBI121, was transformed into tobacco BY-2 cells.

### D. Obtaining an herbicide resistant tobacco strain

Each type of transformed tobacco BY-2 cells (pBI/empty, pBI/Ntpold1^{exo-}, and pBI/ Ntpold1^{exo-}+L567M) were each separately cultured in 100mL liquid suspension medium (NH₄NO₃ 1,650mg/L, KNO₃ 1,900mg/L, CaCl₂-2H₂O 440mg/L, MgSO₄-7H₂O 370mg/L, H₃BO₃ 6.2mg/L, MnSO₄-4H₂O 22.3mg/L, ZnSO₄-7H₂O 8.6mg/L, KI 0.83mg/L, Na₂MoO₄-2H₂O 0.25mg/L, CuSO₄-5H₂O 0.025mg/L, CoCl₂-6H₂O 0.025mg/L, Na₂-EDTA 37.3mg/L, FeSO₄-7H₂O 27.8mg/L, Thiamine-HCl 1.0mg/L, myo-Inositol 100mg/L, Sucrose 30g/L, 2,4-Dichlorophenoxyacetic acid 0.2mg/L) with a 300ml Flask at 27 degree Celsius with shaking at 130rpm. Actively growing cells were inoculated into fresh liquid medium. After four days of growth, approximately 50mL of the transformed cell suspension was transferred to tube and centrifuged at 200xg for 5 minutes at room temperature. The supernatant was removed and pelleted cells were resuspended in fresh liquid medium to rinse the cells. The cells were centrifuged and rinsed three times.

After rinsing, the concentration of the transformed cells in the liquid medium was equalized across each of the pBI/empty, the pBI/Ntpold1^{exo-}, and the pBI/Ntpold1^{exo-} +L567M mutant cell lines. For each of the cell suspensions, eight plates of solid culture medium having 2.5uM 2,6-Dichlorobenzonitrile (DBN) herbicide were prepared and inoculated with 2mL of the cell suspension. The DBN culture plates were left to grow in the dark at 27 degree Celsius. After 1 month of cell culture, the DBN plates were screened for the presence of growing tobacco callus indicating that the transformed tobacco cells had acquired resistance to DBN.

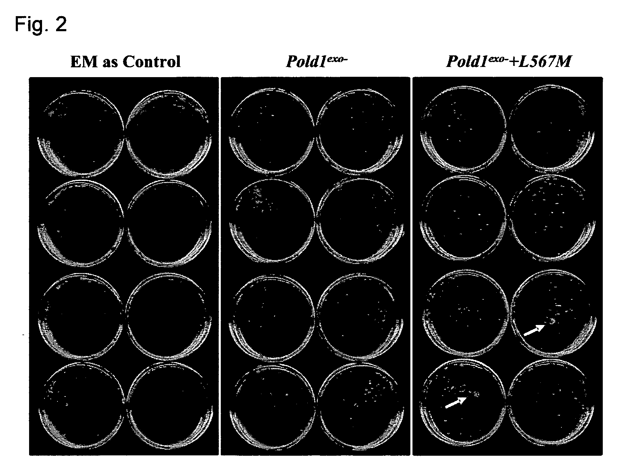

With reference to FIG. 2, after a month of culture, the tobacco cells transformed with the pBI/empty control vector (EM as Control) and the pBI/Ntpold^{exo}-mutant vector did not develop tobacco callus on the DBN plates. However, two of the DBN plates inoculated with the tobacco cells transformed with the pBI/Ntpold1^{exo}+L567M mutant vector (D275A, E277A, and L567M amino acid changes) contained growing tobacco callus (arrows indicate the tobacco callus on the plate). Therefore, the use of a Pold1 mutator gene was successful in directing the evolution of plant cells to develop a desired trait of resistance to the DBN herbicide.

### Example10

### Selecting Organisms with a Desired Trait

Organisms that have been mutated with a mutator gene are screened for a desired trait using screening methods such as those known in the art. In one method of screening for a desired trait, mutated organisms are grown without a specific selective condition. Without a specific selective condition, the organism with the mutator gene accumulates genetic vacation, thereby providing the organism with a desired trait. The organism is then selected that demonstrates the desired trait such as the organism grows more efficiently or better produces a desired product or a new product.

Organisms with a desired trait are also selected by growth under selective conditions. The selective conditions are chosen from physical growth conditions, the presence of certain chemicals, particular biological conditions, or combinations thereof.

The physical growth conditions are selected from particular pH conditions, certain temperatures, desired atmospheric pressures, or other physical conditions and combinations thereof that are experienced during growth of the organism.

Selective chemical conditions are used including the presence of chemical substances such as organic solvents, antibiotics, halogenated compounds, aromatic compounds, analogues, or other chemical compounds or formulas.

Mutated organisms with a desired biological trait are chosen by exposing the organism to selective biological conditions including a high density of the organism or exposing the organism to the presence of other species or types of organisms. Organisms are then selected according to their ability to tolerate high population densities or produce a desired product, such as a pharmaceutical compound, despite high population densities.

Mutated organisms are also screened for the ability to survive or produce a desired product, such as a pharmaceutical compound, in the presence of different species of organisms or pathogens. Organisms that survive under these selective biological conditions do so because they are able to successfully compete for limited resources and/or because they grow synergistically or cooperatively with the surrounding organisms. Those mutated organisms that demonstrate the desired trait may be selected and isolated for further study and use.

The use of mutator genes in combination with genetic modification of mutated organisms allows for the selection of desired traits. Organisms transformed with mutator genes are genetically modified at desired locations in the organisms genome. The genetically modified organisms are screened for desired traits including more efficient growth and the production of a desired product or compound.

### Comparative Example 11

### Construction of a E. coli mutator vector

The DNA polymerase II (POLII) of E. coli is the product of the polB gene (SEQ ID NO: 84). The structures of the POLII exonuclease and polymerase domains are similar to the exonuclease and polymerase domains of eukaryotic POL delta. For construction of E. coli mutator vectors comprising pUC/polII plasmids, PCR products of the wild-type POLII coding region and promoter were inserted into the multi-cloning site of pUC-ori vector. The polB sequences used for the design of PCR primers were, EcpolBEcoU1: GCTTGAATTCGCGCGAAGGCATATTACGGGC (SEQ ID NO: 85) and EcpolBD1: TCAAGCATGCGTACTGGATGGCAAAGCATTCGTC (SEQ ID NO: 86). For the exonuclease-deficient polIII plasmid, D155A (aspartic acid to alanine change at amino acid 155) and E157A (glutamic acid to alanine change at amino acid 157) mutations were created. The pol3-01 type mutations were produced in the POLII coding sequence using a QuikChange Kit (Stratagene), resulting in the polII^{exo-} coding sequence. The 3' to 5' exonuclease region and the polymerase fidelity region of the polII gene were modified to create a mutator gene comprising an L422G (a leucine to glycine change at amino acid 422) mutation in the polII^{exo-} coding sequence using the QuikChange Kit (Stratagene), resulting in the polII^{exo-}+L422G mutator gene coding sequence. Competent E. coli cells (MG1655 strain) were grown and prepared using standard methods. The competent cells were transformed with the pUC/polII^{exo-}or pUC/polII^{exo-}+L422G plasmids to generate pUC/polII^{exo-} or pUC/polII^{exo}+L422G E. coli strains. The endogenous wild-type POLII gene of the transformants remained intact and active

Fluctuation Analysis of Mutation Rates at the Rifampicin resistant locus in E. coli The E. coli transformants with pUC/polII wild type or pUC/polII^{exo-} or pUC/polII^{exo-} +L422G were assayed to determine the mutation rate as measured by the gain of Rifampicin resistance. Each of the transformants was grown in LB liquid medium to the stationary phase. Then, 50 to 100ul of each of the cultures was placed onto LB solid medium including 100ug/ml Rifampicin and incubated at 37 degree Celsius for 18 hours. Mutation rates were determined from the number of the Rifampicin resistant (Rifampicin^{R}) colonies that developed according to the Lea Coulson's index as discussed previously. The fluctuation analysis was repeated five times for each cell culture and the average mutation rates were determined excluding the highest and the lowest numbers. The results are shown in TABLE 5.
[Table 5]

**TABLE 5**

| Mutation rates (MR) at the Rifampicin^{R} locus in *E.coli* | | | | | |
|---|---|---|---|---|---|
| po*III* vectors | 1 | 2 | 3 | Ave. | Relative MR |
| *No vector* | 7.7×10⁻⁹ | 2.0×10⁻⁸ | 1.9×10⁻⁸ | 1.5×10⁻⁸ | 1 |
| *poIII* (wild type) | 6.9×10⁻⁹ | 2.5×10⁻⁸ | 3.6×10⁻⁸ | 2.2×10⁻⁸ | 1.4 |
| *poIII^{exo-}* | 2.9×10⁻⁸ | 3.1×10⁻⁷ | 5.5×10⁻⁷ | 2.9×10⁻⁷ | 19 |
| *poIII^{exo-} +L422G* | 3.5×10⁻⁵ | 7.3×10⁻⁵ | 5.9×10⁻⁶ | 5.5×10⁻⁵ | 3599 |

| | | | | | |
|---|---|---|---|---|---|
| *Mutation rates were determined by fluctuation tests. | | | | | |

The wild type mutation rate at the at the Rifampicin^{R} locus in E. coli without a vector averaged approximately 1.5x10⁻⁸. The mutation rate at the Rifampicin^{R} locus in E. coli with wild-type POLII on a vector averaged approximately 2.2x10⁻⁸, only 1.4 times greater than no vector wild type. The mutation rate of the wild type and that of the wild-type POLII vector were almost the same. The mutation rate of the polII^{exo-} mutator was approximately 2.9x10⁻⁷, which is again only 19 times greater than the no vector wild type. However, the polII^{exo-}+L422G mutator strain had a mutation rate of approximately 5.5x10⁻⁵, over 3500 times greater than the no vector wild type.

### Comparative Example 12

### Construction of a gram-positive bacteria mutator vector

The DNA polymerase III (POLIII) of gram-positive bacteria, for example, Bacilllus. subtilis (B. subtilis) is the product of the polC gene (SEQ ID NO:89). For construction of gram-positive bacteria mutator vectors, EcoRI/BamHI fragment of the wild-type POLIII coding region and promoter are inserted into the multi-cloning site of a plasmid which have a origin of replication active in gram-positive bacteria; for example, pUB 110, pAMalphal, pC194 or pBC16. For the exonuclease-deficient polC plasmid, one or more of the amino acid residue responsible for 3' to 5' exonuclease activity may be changed to an inactivated amino acid residue on, for example, pAMalpha1 plasmid. The amino acid residues responsible for 3' to 5' exonuclease activity are, for example, D425, E427, G430 in ExoI region, and D510 in ExoII region. Changes in these amino acid residues that result in 3' to 5' exonuclease deficiency are, for example, D (acidic amino acid) 425 change to non-acidic amino acid, such as D425A (aspartic acid to alanine change at amino acid 425). Another amino acid change that result in exonuclease deficiency is, for example, E (acidic amino acid) 427 change to non-acidic amino acid such as E427A (glutamic acid to alanine change at amino acid 427), or E427Q (glutamic acid to glutamine change at amino acid 427). Yet another amino acid change that result in 3' to 5' exonuclease deficiency is, for example, G (neutral amino acid) 430 change to non-neutral amino acid such as G430E (glycine to glutamic acid change at amino acid 430). Yet another amino acid change that result in 3' to 5' exonuclease deficiency is, for example, D (acidic amino acid) 510 change to non-acidic amino acid such as D510N (aspartic acid to asparagine change at amino acid 510), D510A (aspartic acid to alanine change at amino acid 510), or D510G (aspartic acid to glycine change at amino acid 510). The mutation is performed in the above POLIII coding sequence using QuikChange Kit (Stratagene) to produce pAMalphal/polCexo-. In order to obtain the polC plasmid with both exonuclease-deficient and reduced fidelity activity, one or more changes in the amino acid residues responsible for polymerase fidelity is inactivated in addition to the above modification in 3' to 5' exonuclease activity. Change in the amino acid residues that result in polymerase fidelity is, for example, S (neutral amino acid) 972 change to non-neutral amino acid such as S972P (serine to proline change at amino acid 972). Another amino acid change that result in reduced polymerase fidelity is, for example, L (neutral amino acid) 1177 change to non-neutral amino acid such as L1177W (leucine to tryptophan change at amino acid 1177). Yet another amino acid change that result in reduced polymerase fidelity is, for example, F (aromatic amino acid) 1264 change to non-aromatic amino acid such as F1264S (phenylalanine to serine change at amino acid 1264). The mutation(s) may be performed using QuikChange Kit (Stratagene), resulting in the pAMalphal/polCexo-&fidelity-. B. subtilis are grown and prepared for the competent cells using standard methods. The competent cells are transformed with the pAMalphal/polCexo- or pAMalphal/polCexo-&fidelity- plasmids to generate pAMalphal/polCexo- or pAMalphal/polCexo-&fidelity- B. subtilis strains. The endogenous wild-type POLIII gene of the transformants is remained intact and active.

### Mutation frequency at Rifampicin resistant locus in B. subtilis

The transformants with pAMalphal/polCexo- or pAMalphal/polCexo-&fidelity- are assayed to determine the mutation rate frequency as measured by the gain of Rifampicin resistance. Five colonies of each of the transformants are grown in LB liquid medium to the stationary phase. Then 10ul of each of the cultures is placed onto LB solid medium including 10ug/ml Rifampicin and incubated at 37 degree Celsius for 18 hours. Mutation frequency is determined from the number of the Rifampicin resistant colonies that developed.

Transformants have a mutation frequency ranging from approximately a 2-fold increase, to at most a 100,000-fold increase in the mutation rate relative to the parent strain. More specifically, the mutator plasmid confers an increased mutation rate of approximately 10, 20, 30, 40, 50, 60, 70, 80, 100, 120, 140, 160, 170, 190, 200, 250, 300, 350, and 400-fold greater than the mutation rate of the parent strain. In other transformants, the mutator gene causes at least 2, 3, 4, 5, 6, 7, 8, 9, or 10 mismatched bases, or at least 15, 20, 25, 50, and 100 mismatched bases per DNA replication.

As will be apparent to those of ordinary skill in the art, many changes may be made to the details of the above-described embodiments without departing from the underlying principles of the invention. The scope of the present invention should, therefore, be determined only by the following claims.

## Claims

1. A method of directing the evolution of a Chinese hamster ovary (CHO) cell, the method comprising:
introducing at least one mutator gene into the CHO cell, wherein the at least one mutator gene comprises a low fidelity mutation and an exonuclease deficient mutation, wherein the introduction of the at least one mutator gene increases the mutation rate of the CHO cell;
growing the CHO cell into which the at least one mutator gene is introduced to obtain a grown cell; and
selecting at least one mutated cell with a desired hereditary trait from the grown cell, wherein
(a) the exonuclease deficient mutation comprises a D398A (aspartic acid to alanine change at amino acid 398) amino acid substitution in at least one catalytic subunit of a DNA polymerase delta gene (Poldl) of the CHO cell; and
(b) the low fidelity mutation comprises a L602M (leucine to methionine change at amino acid 602) amino acid substitution in at least one catalytic subunit of a DNA polymerase delta gene (Poldl) of the CHO cell.

2. The method of claim 1, further comprising restoring a wild-type mutation rate of the grown cell to obtain a restored grown cell,
wherein the at least one mutated cell with a desired hereditary trait is selected from the restored grown cell.

3. The method of claim 1, further comprising restoring a wild-type mutation rate of the selected cell.

## Patentansprüche

1. Verfahren zur Steuerung der Evolution einer CHO-Zelle (Ovarialzelle des chinesischen Hamsters), wobei das Verfahren umfasst:
das Einbringen mindestens eines Mutatorgens in die CHO-Zelle, wobei das mindestens eine Mutatorgen eine Low-Fidelity-Mutation und eine Exonuklease-defiziente Mutation umfasst, wobei das Einbringen des mindestens einen Mutatorgens die Mutationsrate der CHO-Zelle erhöht;
das Kultivieren der CHO-Zelle, in die das mindestens eine Mutatorgen eingebracht ist, zum Erhalt einer gezüchteten Zelle; und
das Selektieren mindestens einer mutierten Zelle mit einem gewünschten Erbmerkmal aus der gezüchteten Zelle, wobei
(a) die Exonuklease-defiziente Mutation eine D398A-Aminosäuresubstitution (Substitution von Asparaginsäure durch Alanin an Aminosäure 398) in mindestens einer katalytischen Untereinheit eines DNA-Polymerase-Delta-Gens (Pold1) der CHO-Zelle umfasst; und
(b) die Low-Fidelity-Mutation eine L602M-Aminosäuresubstitution (Substitution von Leucin durch Methionin an Aminosäure 602) in mindestens einer katalytischen Untereinheit eines DNA-Polymerase-Delta-Gens (Pold1) der CHO-Zelle umfasst.

2. Verfahren nach Anspruch 1, des Weiteren umfassend das Wiederherstellen einer Wildtyp-Mutationsrate der gezüchteten Zelle zum Erhalt einer wiederhergestellten gezüchteten Zelle,
wobei die mindestens eine mutierte Zelle mit einem gewünschten Erbmerkmal aus der wiederhergestellten gezüchteten Zelle selektiert ist.

3. Verfahren nach Anspruch 1, des Weiteren umfassend das Wiederherstellen einer Wildtyp-Mutationsrate der selektierten Zelle.

## Revendications

1. Procédé d'orientation de l'évolution d'une cellule d'ovaire de hamster chinois (CHO), le procédé comprenant :
- l'introduction d'au moins un gène mutateur dans la cellule CHO, le au moins un gène mutateur comprenant une mutation de faible fidélité et une mutation déficiente en exonucléase, l'introduction du au moins un gène mutateur augmentant le taux de mutation de la cellule CHO ;
- la croissance de la cellule CHO dans laquelle le au moins un gène mutateur est introduit pour obtenir une cellule cultivée ; et
- la sélection d'au moins une cellule mutée ayant un caractère héréditaire souhaité à partir de la cellule cultivée :
(a) la mutation déficiente en exonucléase comprenant une substitution d'acide aminé D398A (changement d'acide aspartique en alanine à l'acide aminé 398) dans au moins une sous-unité catalytique d'un gène d'ADN polymérase delta (Pold1) de la cellule CHO ; et
(b) la mutation de faible fidélité comprenant une substitution d'acide aminé L602M (changement de leucine en méthionine à l'acide aminé 602) dans au moins une sous-unité catalytique d'un gène d'ADN polymérase delta (Pold1) de la cellule CHO.

2. Procédé selon la revendication 1, comprenant en outre la restauration d'un taux de mutation de type sauvage de la cellule cultivée pour obtenir une cellule cultivée restaurée,
la au moins une cellule mutée ayant un caractère héréditaire souhaité étant choisie à partir de la cellule cultivée restaurée.

3. Procédé selon la revendication 1, comprenant en outre la restauration d'un taux de mutation de type sauvage de la cellule sélectionnée.


### SEQUENCE LISTING

<110> Neo-Morgan Laboratory Incorporated Ogawa, Masanori
<120> Method of Directing the Evolution of an Organism
<130> 08-180PCT
<150> US61/061,485
   <151> 2008-06-13
<160> 89
<170> Patent In version 3.3
<210> 1
   <211> 4794
   <212> DNA
   <213> Saccharomyces cerevisiae
<400> 1
   tcttcgttca acttgttttc cttgatggca cggtagagtc caccatttcc atctggtgat 60
   tgagataggt ttactgggtc tttcattagg aaatgcttcc cggttaaatc aaaggcaggc 120
   agggttccct ggttgaagaa cgtaatttgt tctttattca agccaaaata attgtgttct 180
   tgaaagtatg cctcagtagc cgctctagtg gggcctgatg tcataatata ccaaggaatt 240
   tctacctttt tgtcctttac catatcttgc aacctgatca acttttcagc ttgaatttga 300
   aaaagagatt tcttagaagg caatccaatg tcgtaacagc cctttggttg agaggatcct 360
   aaccgcgtac cttgtccgcc agccattaaa atcactgcga cttcaccctt gccaatagct 420
   tcaaggccta aacgccagta ttcattttct ttcttactgt tgccaataag cgactcgtac 480
   gaagtagggg gcaatggtga aatttcgacg ccagtatcct tagaagagtt agctagtgag 540
   aatttaatag cattttgaca gtcttccagt agttttgcag gggacctctt ggaagatatt 600
   tgctccaggt ttgaaagcaa ttcttcttgg tctttgcgag acaagctttc ccaattgtgg 660
   aaaagttgac tttgtccggc ttcaatgaat agctgttttg tgtcagtcat agttgcagtt 720
   cgatctggtg ttctccttta tatctgcgtt tctttgcagc gttcttgctt gacggttgat 780
   cttggttttg tgtggtaaaa gtatgcctta acgtaatagg caatttttcg tattacgcgt 840
   aactttttat tctataaaat gttcaatgag gacatctgct attcgcttat gaagaacaaa 900
   cactcagtac tactgatcta aggcaatttt caaggataaa ggaaaataga tattgagcac 960
   ttgctattaa gcattaatct ttatacatat acgcacagca atgagtgaaa aaagatccct 1020
   tcccatggtt gatgtgaaga tcgatgacga ggatactccc cagttggaaa agaaaatcaa 1080
   acggcaatca atagatcatg gtgttggaag tgaacctgtt tcaacaatag agattattcc 1140
   gagtgattct tttcgaaaat ataatagtca aggcttcaaa gcaaaggata cagatttaat 1200
   gggtacgcaa ttagagtcta cttttgaaca agacgtatcg caaatggaac atgatatggc   1260
   cgaccaagaa gagcatgacc tgtcatcatt cgagcgtaag aaacttccaa ccgattttga   1320
   cccaagtttg tatgatattt ctttccaaca aattgatgcg gaacagagcg tactgaatgg   1380
   tatcaaagat gaaaatacat ctaccgtggt aaggtttttt ggtgtcacta gtgaaggaca   1440
   ctctgtactt tgtaatgtta cagggttcaa gaactatctt tacgtcccag cgcccaattc   1500
   ttccgacgct aacgatcagg agcaaatcaa caagtttgtg cactatttaa acgaaacatt   1560
   tgaccacgct attgattcga ttgaagttgt atctaaacag tctatctggg gttattccgg   1620
   agataccaaa ttaccattct ggaaaatata cgtcacctat ccgcatatgg tcaacaaact   1680
   gcgtactgcg tttgaaagag gtcatctttc attcaactcg tggttttcta acggcacgac   1740
   tacttatgat aacattgcct acactttaag gttaatggta gattgtggaa ttgtcggtat   1800
   gtcctggata acattaccaa aaggaaagta ttcgatgatt gagcctaata acagagtttc   1860
   ctcttgtcag ttggaagttt caattaatta tcgtaaccta atagcacatc ctgctgaggg   1920
   tgattggtct catacagctc cattgcgtat catgtccttt gatatcgagt gtgctggtag   1980
   gattggcgtc tttccggaac ctgaatacga tcccgtcatc caaattgcca acgttgtgag   2040
   tattgctggc gctaagaaac cattcattcg taatgtgttt actctgaata catgctcacc   2100
   cataacaggt tcaatgattt tttcccacgc cactgaagag gaaatgttga gcaattggcg   2160
   taactttatc atcaaagttg atcctgatgt tatcattggt tataatacta caaattttga   2220
   tatcccttat cttttaaacc gtgcaaaggc gctaaaggtg aatgatttcc catattttgg   2280
   aaggttaaaa accgttaagc aagaaattaa agagtctgtg ttctcttcga aggcttatgg   2340
   tacaagagaa accaaaaatg tcaatattga cggccgatta cagttggatc ttttgcaatt   2400
   tattcagcgt gagtataaac taagatccta cacgttgaat gcagtctctg cgcacttttt   2460
   aggtgaacag aaggaggatg tacattatag catcatttct gatctacaaa atggcgatag   2520
   tgaaacaaga agaaggttgg ccgtttactg tttgaaagac gcctacctgc ctttaaggct   2580
   tatggaaaaa ctaatggcgt tagttaacta tacagaaatg gctcgtgtta caggtgtgcc   2640
   attttcatat ttactagctc gtggtcaaca aattaaagtt gtttctcaac tatttcgaaa   2700
   gtgcctggag attgatactg tgatacctaa catgcaatct caggcctctg atgaccaata   2760
   tgagggtgcc actgttattg agcctattcg tggttattac gatgtaccga ttgcaacttt   2820
   ggatttcaat tctttatatc caagtattat gatggcgcac aacctatgtt atacaacact   2880
   ttgtaacaaa gctactgtag agagattgaa tcttaaaatt gacgaagact acgtcataac   2940
   acctaatgga gattattttg ttaccacaaa aagaaggcgt ggtatattac caattattct   3000
   ggatgaatta ataagtgcta gaaaacgcgc taaaaaagat ctgagagatg agaaggatcc 3060
   attcaaaaga gatgttttaa atggtagaca attggctttg aagatttcag ctaactctgt 3120
   ctatggtttt acaggagcga cggtgggtaa attgccatgt ttagccattt cttcatctgt 3180
   tactgcttat ggtcgtacca tgattttaaa aactaaaacc gcagtccaag aaaaatattg 3240
   tataaagaat ggttataagc acgatgccgt tgtggtttac ggtgacactg attccgttat 3300
   ggtaaagttt ggtacaacag atttaaagga agctatggat cttggtaccg aagctgccaa 3360
   atatgtctcc actctattca aacatccgat taacttagaa tttgaaaaag catacttccc 3420
   ttaccttttg ataaataaaa agcgttatgc aggtttattc tggactaatc ctgacaagtt 3480
   tgacaagttg gaccaaaaag gccttgcttc tgtccgtcgt gattcctgtt ccttggtttc 3540
   tattgttatg aataaagttt taaagaaaat tttaattgaa agaaatgtag atggtgcttt 3600
   agcttttgtc agagaaacta tcaatgatat tctgcataat agagtagata tttcaaagtt 3660
   gattatatca aagacgttag ccccaaatta cacaaatcca cagccgcacg ccgttttggc 3720
   tgaacgtatg aagaggagag agggcgttgg tccaaatgtt ggtgatcgtg tggactatgt 3780
   cattatcggt ggtaatgata aactttacaa tagagcagaa gatccattat ttgtactaga 3840
   aaacaatatt caagtggatt cgcgctatta tttaactaat caattacaaa atccaatcat 3900
   tagtattgtt gcacctatta ttggcgacaa acaggcgaac ggtatgttcg ttgtgaaatc 3960
   cattaaaatt aacacaggct ctcaaaaagg aggcttgatg agctttatta aaaaagttga 4020
   ggcttgtaaa agttgtaaag gtccgttgag gaaaggtgaa ggccctcttt gttcaaactg 4080
   tctagcaagg tctggagaat tatacataaa ggcattatac gatgtcagag atttagagga 4140
   aaaatactca agattatgga cacaatgcca aaggtgcgct ggtaacttac atagtgaagt 4200
   tttgtgttca aataagaact gtgacatttt ttatatgcgg gttaaggtta aaaaagagct 4260
   gcaggagaaa gtagaacaat taagcaaatg gtaaaaaacg atagggtggc acatcatatt 4320
   aggattaaga aaggctaaca actttttgca tgttggtgga tatatatgta tatataaata 4380
   gatacttgga aaaatccaga ttcaaacaat gtttttgaaa taatgcttct catgtttaga 4440
   ggcaagataa ttctgagtat gttttgggta ttttattgtc agtaatttgt aaacgctgca 4500
   attctaatga gaccgagctt tgttgctgta tgtcattgaa ccaagggtgg ttcaaagctt 4560
   catctatatt gtacctttca tcaggattta ggaccaaaag attggatatt aaatgtagta 4620
   ctgagtcatc gattttatcc cagtacggag agtaaaacgc atacttagct tgtaagatct 4680
   gttccttcaa tgaaggtggc cccaattgat cactgaatgg aggaaaacca cataaacaga 4740
   catataatat gacaccagca ctccaaagat ctacttttga tgtgtatcct ttct 4794
<210> 2
   <211> 33
   <212> DNA
   <213> Artificial
<220>
   <223> Primer
<400> 2
   agtcgagtcg actgttttcc ttgatggcac ggt 33
<210> 3
   <211> 33
   <212> DNA
   <213> Artificial
<220>
   <223> Primer
<400> 3
   agtcgagaat tctggagtgc tggtgtcata tta 33
<210> 4
   <211> 41
   <212> DNA
   <213> Artificial
<220>
   <223> Primer
<400> 4
   gcgtatcatg tcctttgcta tcgcgtgtgc tggtaggatt g 41
<210> 5
   <211> 45
   <212> DNA
   <213> Artificial
<220>
   <223> Primer
<400> 5
   gcaactttgg atttcaattc tgcttatcca agtattatga tggcg 45
<210> 6
   <211> 45
   <212> DNA
   <213> Artificial
<220>
   <223> Primer
<400> 6
   gcaactttgg atttcaattc ttgttatcca agtattatga tggcg 45
<210> 7
   <211> 45
   <212> DNA
   <213> Artificial
<220>
   <223> Primer
<400> 7
   gcaactttgg atttcaattc tgattatcca agtattatga tggcg 45
<210> 8
   <211> 45
   <212> DNA
   <213> Artificial
<220>
   <223> Primer
<400> 8
   gcaactttgg atttcaattc tgagtatcca agtattatga tggcg 45
<210> 9
   <211> 45
   <212> DNA
   <213> Artificial
<220>
   <223> Primer
<400> 9
   gcaactttgg atttcaattc tttctatcca agtattatga tggcg 45
<210> 10
   <211> 45
   <212> DNA
   <213> Artificial
<220>
   <223> Primer
<400> 10
   gcaactttgg atttcaattc tggatatcca agtattatga tggcg 45
<210> 11
   <211> 45
   <212> DNA
   <213> Artificial
<220>
   <223> Primer
<400> 11
   gcaactttgg atttcaattc tcattatcca agtattatga tggcg 45
<210> 12
   <211> 45
   <212> DNA
   <213> Artificial
<220>
   <223> Primer
<400> 12
   gcaactttgg atttcaattc tatatatcca agtattatga tggcg 45
<210> 13
   <211> 45
   <212> DNA
   <213> Artificial
<220>
   <223> Primer
<400> 13
   gcaactttgg atttcaattc taagtatcca agtattatga tggcg 45
<210> 14
   <211> 45
   <212> DNA
   <213> Artificial
<220>
   <223> Primer
<400> 14
   gcaactttgg atttcaattc tatgtatcca agtattatga tggcg 45
<210> 15
   <211> 45
   <212> DNA
   <213> Artificial
<220>
   <223> Primer
<400> 15
   gcaactttgg atttcaattc taattatcca agtattatga tggcg 45
<210> 16
   <211> 45
   <212> DNA
   <213> Artificial
<220>
   <223> Primer
<400> 16
   gcaactttgg atttcaattc tccatatcca agtattatga tggcg 45
<210> 17
   <211> 45
   <212> DNA
   <213> Artificial
<220>
   <223> Primer
<400> 17
   gcaactttgg atttcaattc tcagtatcca agtattatga tggcg 45
<210> 18
   <211> 45
   <212> DNA
   <213> Artificial
<220>
   <223> Primer
<400> 18
   gcaactttgg atttcaattc tcgttatcca agtattatga tggcg 45
<210> 19
   <211> 45
   <212> DNA
   <213> Artificial
<220>
   <223> Primer
<400> 19
   gcaactttgg atttcaattc ttcttatcca agtattatga tggcg 45
<210> 20
   <211> 45
   <212> DNA
   <213> Artificial
<220>
   <223> Primer
<400> 20
   gcaactttgg atttcaattc tacttatcca agtattatga tggcg 45
<210> 21
   <211> 45
   <212> DNA
   <213> Artificial
<220>
   <223> Primer
<400> 21
   gcaactttgg atttcaattc tgtttatcca agtattatga tggcg 45
<210> 22
   <211> 45
   <212> DNA
   <213> Artificial
<220>
   <223> Primer
<400> 22
   gcaactttgg atttcaattc ttggtatcca agtattatga tggcg 45
<210> 23
   <211> 45
   <212> DNA
   <213> Artificial
<220>
   <223> Primer
<400> 23
   gcaactttgg atttcaattc ttactatcca agtattatga tggcg 45
<210> 24
   <211> 33
   <212> DNA
   <213> Artificial
<220>
   <223> Primer
<400> 24
   atactgcaag cttacggatt agaagccgcc gag 33
<210> 25
   <211> 32
   <212> DNA
   <213> Artificial
<220>
   <223> Primer
<400> 25
   ccctatctgc agggggtttt ttctccttga cg 32
<210> 26
   <211> 34
   <212> DNA
   <213> Artificial
<220>
   <223> Primer
<400> 26
   atacgcgtcg acatgagtga aaaaagatcc cttc 34
<210> 27
   <211> 33
   <212> DNA
   <213> Artificial
<220>
   <223> Primer
<400> 27
   gccaccgagc tcgaaggaac agatcttaca agc 33
<210> 28
   <211> 21
   <212> DNA
   <213> Artificial
<220>
   <223> Primer
<400> 28
   acgttgtaaa acgacggcca g 21
<210> 29
   <211> 21
   <212> DNA
   <213> Artificial
<220>
   <223> Primer
<400> 29
   tttacggtga cactgattcc g 21
<210> 30
   <211> 45
   <212> DNA
   <213> Artificial
<220>
   <223> Primer
<400> 30
   ggctataata ttcagaactt tgccctccca tacctcatct cgcgc 45
<210> 31
   <211> 35
   <212> DNA
   <213> Artificial
<220>
   <223> Primer
<400> 31
   ccctggattt ctcctctatg tacccatcca tcatg 35
<210> 32
   <211> 26
   <212> DNA
   <213> Artificial
<220>
   <223> Primer
<400> 32
   gtkcayggvt tgarccmtay ttytac 26
<210> 33
   <211> 21
   <212> DNA
   <213> Artificial
<220>
   <223> Primer
<400> 33
   carrccmgcr tabckcttct t 21
<210> 34
   <211> 15
   <212> PRT
   <213> Saccharomyces cerevisiae
<400> 34
   Ile Met Ser Phe Asp Ile Glu Cys Ala Gly Arg Ile Gly Val Phe
   1               5                   10                  15
<210> 35
   <211> 15
   <212> PRT
   <213> Saccharomyces cerevisiae
<400> 35
   Ile Ile Gly Tyr Asn Thr Thr Asn Phe Asp Ile Pro Tyr Leu Leu
   1               5                   10                  15
<210> 36
   <211> 14
   <212> PRT
   <213> Saccharomyces cerevisiae
<400> 36
   Arg Arg Leu Ala Val Tyr Cys Leu Lys Asp Ala Tyr Leu Pro
   1               5                   10
<210> 37
   <211> 15
   <212> PRT
   <213> Pichia stipitis
<400> 37
   Ile Leu Ser Phe Asp Ile Glu Cys Ala Gly Arg Lys Gly Ile Phe
   1               5                   10                  15
<210> 38
   <211> 15
   <212> PRT
   <213> Pichia stipitis
<400> 38
   Ile Ile Gly Tyr Asn Thr Ala Asn Phe Asp Ile Pro Tyr Leu Leu
   1               5                   10                  15
<210> 39
   <211> 14
   <212> PRT
   <213> Pichia stipitis
<400> 39
   Arg Arg Leu Ala Val Tyr Cys Leu Lys Asp Ala Tyr Leu Pro
   1 5 10
<210> 40
   <211> 15
   <212> PRT
   <213> Schizosaccharomyces pombe
<400> 40
   Ile Met Ser Phe Asp Ile Glu Cys Ala Gly Arg Ile Gly Val Phe
   1 5 10 15
<210> 41
   <211> 15
   <212> PRT
   <213> Schizosaccharomyces pombe
<400> 41
   Leu Ile Gly Tyr Asn Ile Cys Asn Phe Asp Ile Pro Tyr Leu Leu
   1 5 10 15
<210> 42
   <211> 14
   <212> PRT
   <213> Schizosaccharomyces pombe
<400> 42
   Arg Arg Leu Ala Ile Tyr Cys Leu Lys Asp Ala Tyr Leu Pro
   1 5 10
<210> 43
   <211> 15
   <212> PRT
   <213> Tolypocladium inflatum
<400> 43
   Ile Leu Ser Phe Asp Ile Glu Cys Ala Gly Arg Lys Gly Ile Phe
   1 5 10 15
<210> 44
   <211> 15
   <212> PRT
   <213> Tolypocladium inflatum
<400> 44
   Ile Ile Gly Tyr Asn Ile Ala Asn Phe Asp Phe Pro Tyr Leu Leu
   1 5 10 15
<210> 45
   <211> 14
   <212> PRT
   <213> Tolypocladium inflatum
<400> 45
   Arg Arg Leu Ala Leu Tyr Cys Leu Lys Asp Ala Tyr Leu Pro
   1               5                   10
<210> 46
   <211> 15
   <212> PRT
   <213> Nicotiana tabacum
<400> 46
   Ile Leu Ser Phe Asp Ile Glu Cys Ala Gly Arg Lys Gly His Phe
   1               5                   10                  15
<210> 47
   <211> 15
   <212> PRT
   <213> Nicotiana tabacum
<400> 47
   Ile Ile Gly Tyr Asn Ile Cys Asn Phe Asp Leu Pro Tyr Leu Leu
   1               5                   10                  15
<210> 48
   <211> 14
   <212> PRT
   <213> Nicotiana tabacum
<400> 48
   Arg Arg Leu Ala Val Tyr Cys Leu Lys Asp Ala Tyr Leu Pro
   1               5                   10
<210> 49
   <211> 15
   <212> PRT
   <213> Arabidopsis thaliana
<400> 49
   Val Leu Ser Phe Asp Ile Glu Cys Ala Gly Arg Lys Gly Ile Phe
   1               5                   10                  15
<210> 50
   <211> 15
   <212> PRT
   <213> Arabidopsis thaliana
<400> 50
   Ile Ile Gly Tyr Asn Ile Cys Lys Phe Asp Leu Pro Tyr Leu Ile
   1               5                   10                  15
<210> 51
   <211> 14
   <212> PRT
   <213> Arabidopsis thaliana
<400> 51
   Arg Arg Leu Ala Val Tyr Cys Leu Lys Asp Ala Tyr Leu Pro
   1               5                   10
<210> 52
   <211> 15
   <212> PRT
   <213> Cricetulus griseus
<400> 52
   Val Leu Ser Phe Asp Ile Glu Cys Ala Gly Arg Lys Gly Ile Phe
   1               5                   10                  15
<210> 53
   <211> 15
   <212> PRT
   <213> Cricetulus griseus
<400> 53
   Ile Thr Gly Tyr Asn Ile Gln Asn Phe Asp Leu Pro Tyr Leu Ile
   1               5                   10                  15
<210> 54
   <211> 14
   <212> PRT
   <213> Cricetulus griseus
<400> 54
   Arg Arg Leu Ala Val Tyr Cys Leu Lys Asp Ala Phe Leu Pro
   1               5                   10
<210> 55
   <211> 15
   <212> PRT
   <213> Mus musculus
<400> 55
   Val Leu Ser Phe Asp Ile Glu Cys Ala Gly Arg Lys Gly Ile Phe
   1               5                   10                  15
<210> 56
   <211> 15
   <212> PRT
   <213> Mus musculus
<400> 56
   Ile Thr Gly Tyr Asn Ile Gln Asn Phe Asp Leu Pro Tyr Leu Ile
   1 5 10 15
<210> 57
   <211> 14
   <212> PRT
   <213> Mus musculus
<400> 57
   Arg Arg Leu Ala Val Tyr Cys Leu Lys Asp Ala Phe Leu Pro
   1 5 10
<210> 58
   <211> 15
   <212> PRT
   <213> Homo sapiens
<400> 58
   Val Leu Ser Phe Asp Ile Glu Cys Ala Gly Arg Lys Gly Ile Phe
   1 5 10 15
<210> 59
   <211> 15
   <212> PRT
   <213> Homo sapiens
<400> 59
   Ile Thr Gly Tyr Asn Ile Gln Asn Phe Asp Leu Pro Tyr Leu Ile
   1 5 10 15
<210> 60
   <211> 14
   <212> PRT
   <213> Homo sapiens
<400> 60
   Arg Arg Leu Ala Val Tyr Cys Leu Lys Asp Ala Tyr Leu Pro
   1 5 10
<210> 61
   <211> 15
   <212> PRT
   <213> Escherichia coli
<400> 61
   Trp Val Ser Ile Asp Ile Glu Thr Thr Arg His Gly Glu Leu Tyr
   1 5 10 15
<210> 62
   <211> 15
   <212> PRT
   <213> Escherichia coli
<400> 62
   Ile Ile Gly Trp Asn Val Val Gln Phe Asp Leu Arg Met Leu Gln
   1               5                   10                  15
<210> 63
   <211> 14
   <212> PRT
   <213> Escherichia coli
<400> 63
   Pro Ala Leu Ala Thr Tyr Asn Leu Lys Asp Cys Glu Leu Val
   1               5                   10
<210> 64
   <211> 12
   <212> PRT
   <213> Saccharomyces cerevisiae
<400> 64
   Ala Thr Leu Asp Phe Asn Ser Leu Tyr Pro Ser Ile
   1               5                   10
<210> 65
   <211> 22
   <212> PRT
   <213> Saccharomyces cerevisiae
<400> 65
   Val Leu Asn Gly Arg Gln Leu Ala Leu Lys Ile Ser Ala Asn Ser Val
   1               5                   10                  15
   Tyr Gly Phe Thr Gly Ala
               20
<210> 66
   <211> 12
   <212> PRT
   <213> Pichia stipitis
<400> 66
   Ala Thr Leu Asp Phe Ser Ser Leu Tyr Pro Ser Ile
   1               5                   10
<210> 67
   <211> 22
   <212> PRT
   <213> Pichia stipitis
<400> 67
   Val Leu Asn Gly Arg Gln Leu Ala Leu Lys Ile Ser Ala Asn Ser Val
   1               5                   10                  15
   Tyr Gly Phe Thr Gly Ala
               20
<210> 68
   <211> 12
   <212> PRT
   <213> Schizosaccharomyces pombe
<400> 68
   Ala Thr Leu Asp Phe Ser Ser Leu Tyr Pro Ser Ile
   1               5                   10
<210> 69
   <211> 22
   <212> PRT
   <213> Schizosaccharomyces pombe
<400> 69
   Val Leu Asp Gly Arg Gln Leu Ala Leu Lys Val Ser Ala Asn Ser Val
   1               5                   10                  15
   Tyr Gly Phe Thr Gly Ala
               20
<210> 70
   <211> 12
   <212> PRT
   <213> Tolypocladium inflatum
<400> 70
   Ala Thr Leu Asp Phe Ala Ser Leu Tyr Pro Ser Ile
   1               5                   10
<210> 71
   <211> 22
   <212> PRT
   <213> Tolypocladium inflatum
<400> 71
   Val Leu Asn Gly Arg Gln Leu Ala Leu Lys Ile Ser Ala Asn Ser Val
   1               5                   10                  15
   Tyr Gly Leu Thr Gly Ala
               20
<210> 72
   <211> 12
   <212> PRT
   <213> Nicotiana tabacum
<400> 72
   Ala Thr Leu Asp Phe Ala Ser Leu Tyr Pro Ser Ile
   1               5                   10
<210> 73
   <211> 22
   <212> PRT
   <213> Nicotiana tabacum
<400> 73
   Val Leu Asp Gly Arg Gln Leu Ala Leu Lys Ile Ser Ala Asn Ser Val
   1               5                   10                  15
   Tyr Gly Phe Thr Gly Ala
               20
<210> 74
   <211> 12
   <212> PRT
   <213> Arabidopsis thaliana
<400> 74
   Ala Thr Leu Asp Phe Ala Ser Leu Tyr Pro Ser Ile
   1               5                   10
<210> 75
   <211> 22
   <212> PRT
   <213> Arabidopsis thaliana
<400> 75
   Val Leu Asp Gly Arg Gln Leu Ala Leu Lys Ile Ser Ala Asn Ser Val
   1               5                   10                  15
   Tyr Gly Phe Thr Gly Ala
               20
<210> 76
   <211> 12
   <212> PRT
   <213> Cricetulus griseus
<400> 76
   Ala Thr Leu Asp Phe Ser Ser Leu Tyr Pro Ser Ile
   1               5                   10
<210> 77
   <211> 22
   <212> PRT
   <213> Cricetulus griseus
<400> 77
   Val Leu Asp Gly Arg Gln Leu Ala Leu Lys Val Ser Ala Asn Ser Val
   1               5                   10                  15
   Tyr Gly Phe Thr Gly Ala
               20
<210> 78
   <211> 12
   <212> PRT
   <213> Mus musculus
<400> 78
   Ala Thr Leu Asp Phe Ser Ser Leu Tyr Pro Ser Ile
   1               5                   10
<210> 79
   <211> 22
   <212> PRT
   <213> Mus musculus
<400> 79
   Val Leu Asp Gly Arg Gln Leu Ala Leu Lys Val Ser Ala Asn Ser Val
     1               5                   10                  15
   Tyr Gly Phe Thr Gly Ala
               20
<210> 80
   <211> 12
   <212> PRT
   <213> Homo sapiens
<400> 80
   Ala Thr Leu Asp Phe Ser Ser Leu Tyr Pro Ser Ile
   1               5                   10
<210> 81
   <211> 22
   <212> PRT
   <213> Homo sapiens
<400> 81
   Val Leu Asp Gly Arg Gln Leu Ala Leu Lys Val Ser Ala Asn Ser Val
   1 5 10 15
   Tyr Gly Phe Thr Gly Ala
   20
<210> 82
   <211> 12
   <212> PRT
   <213> Escherichia coli
<400> 82
   Leu Val Leu Asp Tyr Lys Ser Leu Tyr Pro Ser Ile
   1 5 10
<210> 83
   <211> 22
   <212> PRT
   <213> Escherichia coli
<400> 83
   Gly Asn Lys Pro Leu Ser Gln Ala Leu Lys Ile Ile Met Asn Ala Phe
   1 5 10 15
   Tyr Gly Val Leu Gly Thr
   20
<210> 84
   <211> 2352
   <212> DNA
   <213> Escherichia coli
<400> 84
<210> 85
   <211> 31
   <212> DNA
   <213> Escherichia coli
<400> 85
   gcttgaattc gcgcgaaggc atattacggg c 31
<210> 86
   <211> 34
   <212> DNA
   <213> Escherichia coli
<400> 86
   tcaagcatgc gtactggatg gcaaagcatt cgtc 34
<210> 87
   <211> 3312
   <212> DNA
   <213> Cricetulus griseus
<400> 87
   atggatttta agcggcgaca aggaccaggc cctggggtgc ccccaaaacg ggctcgtggg 60
   ggtctctggg atgaggacga gccatcgcaa tttgaggaga acttggcgtt gctggaggag 120
   atagaggctg agaatcggct gcaggaggca gaggaggagc tgcagctgcc cccacagggc 180
   cctgcaggtg ggcagttttc cactgcagac attgacccac gctggaagcg gcccgccctg 240
   tgtgccctgg atcccaacac ggagcccctc atcttccagc agctagagat tgaccactat 300
   gttggctcag ctgtgccact gccaggagga cccccaacgt cctgtaattc agtgcccata 360
   ctacgggcct ttggggtgac tgatgagggc ttctccgtct gctgccatat ccatggcttt    420
   gcaccctact tctacacccc tgcacctccc ggtttcgggg ccgagcacct gagtgacctg    480
   cagcgagagc tgagcacagc catcagccgg gaccagcgtg gtgggaagga gctgtcgggg    540
   ccagcagtgc tggccataga gctgtgctcc cgtgagagca tgtttgggta ccatggtcac    600
   ggcccctctc catttctccg catcaccctg gcgctacccc gccttgtggc accagcccgc    660
   cgcctcctgg aacagggtat ccgtgtgcca ggcctgggta ccccaagctt cgcaccctat    720
   gaggccaacg tggactttga gatccggttc atggtggatg ctgacatcgt gggctgcaac    780
   tggttggagc tcccagctgg gaagtatgtt tggaggacag agaagaaggc tacacagtgc    840
   cagctggagg tggatgtgtt gtggtcagat gtcatcagtc acccaccaga agggcagtgg    900
   cagcgtattg cgccccttcg tgtacttagc tttgacatcg agtgtgctgg ccgcaaaggc    960
   atcttccctg agccggagcg tgaccctgtg atccagatct gttccctggg gctgcggtgg   1020
   ggagaaccag agccgttctt gcgcctggca ctcacactga ggccctgtgc acccatcctt   1080
   ggtgccaaag tgcagagcta tgagcgggaa gaagacctgc tccaggcctg ggccaacttc   1140
   atccttgcca tggatcctga tgtgataact ggctataata ttcagaactt tgacctccca   1200
   tacctcatct cgcgcgcgca aaccctgaag gtggaccgct tccctttcct gggccgagtg   1260
   actggtctcc gctccaacat ccgagattcc tccttccagt cgaggcaggt gggccggcgg   1320
   gacagtaagg tggtcagcat ggtgggtcga gttcagatgg acatgctgca ggtgctgctg   1380
   cgggagcaca agctgcgctc ctacaccctg aatgctgtca gcttccactt cctgggtgag   1440
   cagaaggagg acgtgcagca cagcatcatc accgacctgc agaacgggaa tgagcagaca   1500
   cgccgccgcc tggccgtgta ctgcctgaag gacgccttcc tgccacttcg gctgcttgag   1560
   cgccttatgg tgctggtgaa caatgtagaa atggcgcgtg tcacgggtgt gccccttgga   1620
   tacctgctca gccggggcca acaggtcaag gtcgtgtctc agctgctgcg ccaggccatg   1680
   cgccaggggc tcctgatgcc tgtggtgaag actgagggcg gtgaggacta cacgggagcc   1740
   actgtcattg agccccttaa agggtactat gacgtcccca ttgccaccct ggatttctcc   1800
   tctctgtacc catccatcat gatggcccat aatctgtgct acaccacact gctacggccc   1860
   ggggctgccc agaagttggg ccttaaacca gatgagttca tcaagacacc cactggagat   1920
   gagtttgtga agtcatctgt gcggaagggc ctcctgcccc agatcctgga gaatctgctg   1980
   agtgcccgga agagggccaa ggctgagctg gctcaggaga cagacccctt gcggcggcag   2040
   gtcttggatg gacggcagct ggcactgaaa gtgagtgcca actctgtgta tggcttcact   2100
   ggtgcccagg tgggcaagct gccatgtttg gagatctctc agagtgtcac tgggttcggg   2160
   cggcagatga ttgagaaaac caagcaacta gtggagtcca agtacaccct ggaaaatggc   2220
   tacaatgcca atgccaaggt ggtctatggt gacactgact ctgtgatgtg ccgatttggt   2280
   gtctcctctg tggctgaggc aatgtctctg ggtcgagagg ctgcaaactg ggtatctagt   2340
   cacttcccat cacccatccg actggagttt gagaaggttt acttcccata cctgctcatc   2400
   agcaagaagc gctatgctgg cctgctcttc tcctcccagc ctgataccca tgacagaatg   2460
   gactgcaagg gcctggaggc tgtgcgcagg gacaactgtc ccctggtggc caaccttgtt   2520
   acatcctctc tgcgacgaat ccttgtggac cgggatcccg atggggcagt ggcccatgca   2580
   aaggatgtga tctcagacct gctgtgtaac cgcatagaca tctcccagct ggtcatcacc   2640
   aaggagctga cccgagcagc cgcagactat gctggcaagc aggctcatgt ggaactggct   2700
   gagaggatga ggaagcgtga ccccggcagt gcgcccagct tgggtgaccg cgtcccctat   2760
   gtgatcatcg gtgctgccaa aggcgtggct gcctacatga agtcggagga ccccctgttt   2820
   gtgctggaac acagcctgcc catcgacact caatactacc tggagcagca gctggccaag   2880
   ccactgctgc gaatttttga gcccattctg ggtgagggcc gtgcagagtc tgtgctgctc   2940
   cgaggagacc acacacgctg caaaaccgtg ctcaccagca aggttggtgg cctcctagcc   3000
   ttcaccaagc gccgcaactg ttgcattggc tgccgctcag tgatcaacca tcagggagct   3060
   gtgtgtgaat tctgtcagcc ccgggagtcg gagctctatc agaaggaggt gtcacacctg   3120
   aatgccttgg aggaacgctt ctcgcgcctc tggacacagt gccagcgttg ccagggcagc   3180
   ttgcatgagg acgtcatctg caccagccgc gactgtccca tcttctacat gcgcaaaaaa   3240
   gtgcgcaagg acctggagga ccaggagcgg ctgctgcagc gttttggacc tccgggaccc   3300
   gaggcttggt ga                                                        3312
<210> 88
   <211> 3180
   <212> DNA
   <213> Nicotiana tabacum
<400> 88
   atggaagaag aagacctaga cgatgatgtt ttcctcgaag aaacattgct tcagtacgaa     60
   gaagattccc aaatcctccg tgaaatcgaa gaccgccacg ctctaaatga gcggcttcag    120
   aaatggaaac gccctcctct ttctcaagct tacctctctc agtcccaaag catatcgttt    180
   cagcagctgg agattgacta cattattgga gagagccata aagaattgct gcctaattct    240
   tctggtccag ctgccatttt aagaatcttt ggggtcacca aggaaggaaa tagtgtttgt    300
   tgtcatgtac atggatttga accatacttc tacatcagct gccctcctgg aatgggccct    360
   gacgatatct cccgttttca ccaaattcta gagggtagaa tgagagaggt gaataggaac    420
   agcaatgtcg ctaaatttgt ccgacgcatc gaagtagttc agagaaaaag catcatgcat    480
   taccaaaagc aggcttctca gccttttctc aagattgtag tggcactgcc aacaatggtt     540
   accagttgcc gcggtatcct tgatagaggt attcaaattg atgggcttgg tatgaagagc     600
   tttatgacct atgaaagtaa tgttcttttt gctcttcgct tcatgattga ctgcaacatc     660
   gttggtggca actggattga agttccagtt ggaaaacata agaaagctgc aaggaatctc     720
   tcctatagtc aattagagtt tgattgcctc tattctgact tgatcagcca tcctcctgaa     780
   ggagaatttt caaagatggc tccctttcgc atattgagtt ttgatattga atgtgctggc     840
   cgtaagggcc actttcctga gcctacacaa gatcctgtta ttcaggtggc caatctagtt     900
   accttgcagg gacaggatca gccgttaatt cgtaacgtga tgacccttaa gtcatgctca     960
   cctatagttg gggttgatgt tatgtcattt gacacagaaa aagaagtatt gctggcatgg    1020
   agggatttta tacgggaagt tgaccctgat atcataattg gatataacat ctgcaacttt    1080
   gatttgccat atctcatccg gagagctgaa gtcttaggga tggcagaatt tcctgtgcta    1140
   ggacgcatca gaaacagcag ggttcgtgta aaagattcaa ccttttcttc aagacagcac    1200
   ggcacaaggg aaagtaaaga aataacaata gaagggagag ttcaatttga tttatttcag    1260
   gctgtgcaga gggatcataa acttagttct tactcactga attctgtctc agcacatttt    1320
   cttaatgaac agaaggagga tgtccatcat tcaattatat cagatcttca aaatgggaac    1380
   tcagaaacta ggaggcggct tgctgtgtat tgtctaaagg acgcttatct cccgcaacgg    1440
   cttttggata aattaatgtt catttataac tatgtggaga tggctcgagt cactggtgta    1500
   cctatatctt ttcttctcgc aagagggcaa agcattaagg tactctctca acttttaagg    1560
   aaggcgaggc aaaggaatct tgttttacca aatgtaaaac aggctggatc tgagcaagga    1620
   acatatgagg gtgcgacggt tttggaggcc cgagctggtt tttatgagaa gcctattgca    1680
   actttggatt ttgcatctct atatccgtcg atcatgatgg catataattt atgttactgc    1740
   acgctagtaa ctcctgaaga gttccacaaa ctaaacctcc gtgaagtgga tgtcaacaag    1800
   accccttctg gtgaaatgtt tgtcaagtca gatttacaaa aggggatact tccagaaatt    1860
   cttgaagagc tattggctgc tcgcaaaaga gcaaaagcag atttaaagga agcaaaagat    1920
   cctctagtca aagctgtctt agatggacgt cagctagctt tgaagattag tgcaaattca    1980
   gtatatgggt ttactggagc tactgtcggt caattaccat gtttagaaat atcttcaagt    2040
   gttactagct atggtcgaca gatgattgag aagacaaaaa agcttgtgga ggacaagttc    2100
   acagtactta aggggtatga acacaatgct gaggttatat atggagatac agattcggtg    2160
   atggtgcagt ttggtgtgcc tactgtggaa gaggctatga aattgggtag agaagctgct    2220
   gactacatta gcgagacatt tatcaaacca ataagacttg aatttgagaa gatatattat    2280
   ccatatctgc tgattagcaa gaagagatat gctggccttc tatggacaaa tccagacaaa 2340
   catgacaaaa tggacgctaa agggattgaa actgttagga gggataattg tctcttggtc 2400
   aagaaccttg taaccgagtg ccttcataaa atactaatgg acagagatgt tcctggtgct 2460
   gttcaatatg tcaagaatac tatctcagat cttctcatga accgcatgga tttatctctt 2520
   ttggttatta ctaagggtct gacaaaaacg ggagatgatt atgaagtaaa ggcagcacat 2580
   gttgaacttg cagagcgaat gcgcaagcga gatgctgcta ctgctcctca tattggtgat 2640
   agagtacctt acgtcatcat taaagctgca aaaggttcca aggcatatga aaagtcagag 2700
   gaccctattt acgtattaga gaacaacata cctatagatc ctcattacta tcttgaaaat 2760
   caaattagta agccccttct gaggattttt gagccaatac tgaagaatgc aagtaaagag 2820
   cttcttcaag gaagtcacac aagatcgatc tcaatatcga ccccttcaaa cagtggtatt 2880
   atgaaatttg ctaagaagca actcacttgc attggatgca aagcattaat cagtggttct 2940
   gatagaactc tgtgcaatca ctgcaaagga agagaagctg aactttattg cagatccgta 3000
   actaatgtgg ctgaactaga gaagcttttt gggaggctat ggacacaatg ccaagaatgc 3060
   caaggctctc tccatcagga tgtcctatgc actagtcggg actgcccaat attttatcgg 3120
   aggaagaagg cccagaaaga catggctgaa gccaaacttc aattggatcg gtggaacttt 3180
<210> 89
   <211> 4314
   <212> DNA
   <213> Bacillus subtilis
<400> 89